# EUROPEAN PATENT APPLICATION

(11) **EP 4 183 801 A1**
(43) Date of publication of application: **24.05.2023**
(21) Application number: 21209549.1
(22) Date of filing: 22.11.2021
(51) Int. Cl.: C07K 16/10, A61P 31/14, A61K 39/395

(54) **VHH ANTIBODIES AGAINST SARS-COV-2 VARIANTS**

(71) Applicant: Max-Planck-Gesellschaft zur Förderung der Wissenschaften e.V., 80539 München (DE)
(72) Inventor: The designation of the inventor has not yet been filed
(74) Representative: Weiss, Wolfgang

(57) **Abstract**

The present invention pertains in the fields of antibody technology, protein engineering, medicine, pharmacology, infection biology, virology, and medical diagnostics. More specifically, the present disclosure provides VHH antibodies that prevent cell entry of and infection by SARS-CoV-2 and that have been selected for potent cross-reaction and cross-neutralization between the original Wuhan strain and the Alpha, Beta, Gamma, Delta, and Mu variants of concern.

SEQUENCE LISTING

<110>  Max-Planck Gesellschaft zur Förderung der Wissenschaften
       e.V.

<120>  VHH antibodies against SARS-CoV-2-variants

<130>  202645PEP

<160>  117

<170>  PatentIn version 3.5

<210>  1
<211>  127
<212>  PRT
<213>  Artificial Sequence

<220>
<223>  Re5D06

<400>  1

Gln Val Gln Leu Val Glu Ser Gly Gly Gly Leu Val Gln Pro Gly Gly
1               5                   10                  15

Ser Leu Arg Leu Ser Cys Ala Ala Ser Gly Ile Thr Leu Asp Tyr Tyr
            20                  25                  30

Ala Ile Gly Trp Phe Arg Gln Ala Pro Gly Lys Glu Arg Glu Gly Val
        35                  40                  45

Ser Arg Ile Arg Ser Ser Asp Gly Ser Thr Asn Tyr Ala Asp Ser Val
    50                  55                  60

Lys Gly Arg Phe Thr Met Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Tyr Gly Pro Leu Thr Lys Tyr Gly Ser Ser Trp Tyr Trp Pro Tyr
            100                 105                 110

Glu Tyr Asp Tyr Trp Gly Gln Gly Thr Gln Val Thr Val Ser Ser
        115                 120                 125

<210>  2
<211>  9
<212>  PRT
<213>  Artificial Sequence

<220>
<223>  Re5D06 CDR1

<400>  2

Ile Thr Leu Asp Tyr Tyr Ala Ile Gly
1               5

<210>  3
<211>  15
<212>  PRT
<213>  Artificial Sequence

<220>
<223>  Re5D06 CDR2

<400>  3

Ser Arg Ile Arg Ser Ser Asp Gly Ser Thr Asn Tyr Ala Asp Ser
1               5                   10                  15

<210>  4
<211>  19
<212>  PRT
<213>  Artificial Sequence

<220>
<223>  Re5D06 CDR3

<400>  4

Tyr Gly Pro Leu Thr Lys Tyr Gly Ser Ser Trp Tyr Trp Pro Tyr Glu
1               5                   10                  15

Tyr Asp Tyr

<210>  5
<211>  127
<212>  PRT
<213>  Artificial Sequence

<220>
<223>  Re5D06R15

<400>  5

Gln Val Gln Leu Val Glu Ser Gly Gly Gly Leu Val Gln Pro Gly Gly
1               5                   10                  15

Ser Leu Arg Leu Ser Cys Ala Ala Ser Gly Ile Thr Leu Asp Tyr Tyr
            20                  25                  30

Ala Met Gly Trp Phe Arg Glu Ala Pro Gly Lys Glu Arg Glu Gly Val
        35                  40                  45

Ala Arg Ile Arg Asn Ser Asp Gly Ser Thr Asn Tyr Ala Asp Ser Val
    50                  55                  60

Lys Gly Arg Phe Thr Met Ser Arg Asp Asn Ala Lys Asp Thr Tyr Tyr
65                  70                  75                  80

Leu Gln Met Asn Ser Leu Glu Pro Glu Asp Thr Ala Asp Tyr Tyr Cys
                85                  90                  95

Ala Tyr Gly Pro Leu Thr Lys Tyr Gly Ser Ser Trp Tyr Trp Pro Tyr
            100                 105                 110

Glu Tyr Asp Tyr Trp Gly Gln Gly Thr Gln Val Thr Val Ser Ser
        115                 120                 125

<210>  6
<211>  9
<212>  PRT
<213>  Artificial Sequence

<220>
<223>  Re5D06R15 CDR1

<400>  6

Ile Thr Leu Asp Tyr Tyr Ala Met Gly
1               5

<210>  7
<211>  15
<212>  PRT
<213>  Artificial Sequence

<220>
<223>  Re5D06R15 CDR2

<400>  7

Ala Arg Ile Arg Asn Ser Asp Gly Ser Thr Asn Tyr Ala Asp Ser
1               5                   10                  15

<210>  8
<211>  19
<212>  PRT
<213>  Artificial Sequence

<220>
<223>  Re5D06R15 CDR3

<400>  8

Tyr Gly Pro Leu Thr Lys Tyr Gly Ser Ser Trp Tyr Trp Pro Tyr Glu
1               5                   10                  15

Tyr Asp Tyr

<210>  9
<211>  130
<212>  PRT
<213>  Artificial Sequence

<220>
<223>  Re6H06

<400>  9

Gln Val Gln Leu Val Glu Ser Gly Gly Gly Leu Val Gln Pro Gly Gly
1               5                   10                  15

Ser Leu Arg Leu Ser Cys Ala Ala Ser Gly Val Thr Leu Asp Tyr Tyr
            20                  25                  30

Ala Ile Gly Trp Phe Arg Gln Ala Pro Gly Lys Glu Arg Glu Gly Val
        35                  40                  45

Ser Cys Thr Ser Ser Ser Asp Gly Ser Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Gly Val Tyr Tyr Cys
                85                  90                  95

Ala Val Val Pro Gln Thr Tyr Tyr Gly Gly Lys Tyr Tyr Ser Gln Cys
            100                 105                 110

Thr Ala Asn Gly Met Asp Tyr Trp Gly Lys Gly Thr Leu Val Thr Val
        115                 120                 125

Ser Ser
    130

<210>  10
<211>  9
<212>  PRT
<213>  Artificial Sequence

<220>
<223>  Re6H06 CDR1

<400>  10

Val Thr Leu Asp Tyr Tyr Ala Ile Gly
1               5

<210>  11
<211>  15
<212>  PRT

<213>  Artificial Sequence

<220>
<223>  Re6H06 CDR2

<400>  11

Ser Cys Thr Ser Ser Ser Asp Gly Ser Thr Tyr Tyr Ala Asp Ser
1               5                   10                  15


<210>  12
<211>  22
<212>  PRT
<213>  Artificial Sequence

<220>
<223>  Re6H06 CDR3

<400>  12

Val Val Pro Gln Thr Tyr Tyr Gly Gly Lys Tyr Tyr Ser Gln Cys Thr
1               5                   10                  15


Ala Asn Gly Met Asp Tyr
            20


<210>  13
<211>  130
<212>  PRT
<213>  Artificial Sequence

<220>
<223>  Re9B09

<400>  13

Gln Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Leu Asp Tyr Tyr
            20                  25                  30


Ala Ile Gly Trp Phe Arg Gln Ala Pro Gly Lys Glu Arg Glu Gly Val
        35                  40                  45


Ser Arg Ile Ser Ser Ser Asp Gly Ser Thr Asp Tyr Ala Asp Ser Val
    50                  55                  60


Lys Gly Arg Phe Thr Ile Ser Arg Asp Asn Ala Lys Asn Thr Val Tyr
65                  70                  75                  80


Leu Gln Met Asn Ser Leu Lys Pro Glu Asp Thr Ala Val Tyr Tyr Cys
                85                  90                  95

Ala Thr Val Pro Gly Thr Tyr Tyr Ser Gly Asn Trp Tyr Tyr Thr Trp
            100                 105                 110

His Pro Glu Ala Val Asp Tyr Trp Gly Lys Gly Thr Gln Val Thr Val
        115                 120                 125

Ser Ser
    130

<210>  14
<211>  9
<212>  PRT
<213>  Artificial Sequence

<220>
<223>  Re9B09 CDR1

<400>  14

Phe Thr Leu Asp Tyr Tyr Ala Ile Gly
1               5

<210>  15
<211>  15
<212>  PRT
<213>  Artificial Sequence

<220>
<223>  Re9B09 CDR2

<400>  15

Ser Arg Ile Ser Ser Ser Asp Gly Ser Thr Asp Tyr Ala Asp Ser
1               5                   10                  15

<210>  16
<211>  22
<212>  PRT
<213>  Artificial Sequence

<220>
<223>  Re9B09 CDR3

<400>  16

Thr Val Pro Gly Thr Tyr Tyr Ser Gly Asn Trp Tyr Tyr Thr Trp His
1               5                   10                  15

Pro Glu Ala Val Asp Tyr
            20

<210>  17
<211>  130
<212>  PRT
<213>  Artificial Sequence

<220>
<223>  Re31A08

<400>  17

Gln Val Gln Leu Val Glu Ser Gly Gly Gly Leu Val Gln Pro Gly Gly
1               5                   10                  15

Ser Leu Arg Leu Ser Cys Ala Ala Ser Gly Ile Thr Leu Asp Tyr Tyr
            20                  25                  30

Ser Ile Gly Trp Phe Arg Gln Ala Pro Gly Lys Glu Arg Glu Gly Val
        35                  40                  45

Ser Cys Thr Ser Ser Arg Asp Asp Ser Thr Asp Tyr Ala Asp Ser Val
    50                  55                  60

Lys Gly Arg Phe Thr Ile Ser Arg Asp Asn Ala Lys Asn Ser Val Tyr
65                  70                  75                  80

Leu Gln Met Asn Ser Leu Asn Pro Glu Asp Thr Ala Val Tyr Tyr Cys
                85                  90                  95

Ala Leu Thr Pro Ala Thr Tyr Tyr Ser Gly Arg Tyr Tyr Phe Gln Cys
            100                 105                 110

Pro Pro His Gly Met Asp Tyr Trp Gly Lys Gly Thr Leu Val Thr Val
        115                 120                 125

Ser Ser
    130

<210>  18
<211>  9
<212>  PRT
<213>  Artificial Sequence

<220>
<223>  Re31A08 CDR1

<400>  18

Ile Thr Leu Asp Tyr Tyr Ser Ile Gly
1               5

<210>  19
<211>  15
<212>  PRT
<213>  Artificial Sequence

<220>
<223>  Re31A08 CDR2

<400>  19

Ser Cys Thr Ser Ser Arg Asp Asp Ser Thr Asp Tyr Ala Asp Ser
1               5                   10                  15


<210>  20
<211>  22
<212>  PRT
<213>  Artificial Sequence

<220>
<223>  Re31A08 CDR3

<400>  20

Leu Thr Pro Ala Thr Tyr Tyr Ser Gly Arg Tyr Tyr Phe Gln Cys Pro
1               5                   10                  15


Pro His Gly Met Asp Tyr
            20


<210>  21
<211>  130
<212>  PRT
<213>  Artificial Sequence

<220>
<223>  Re31A12

<400>  21

Gln Val Gln Leu Val Glu Ser Gly Gly Gly Leu Val Gln Ser Gly Gly
1               5                   10                  15


Ser Leu Arg Leu Ser Cys Leu Thr Ser Gly Val Thr Leu Asp Tyr Tyr
            20                  25                  30


Ala Ile Gly Trp Phe Arg Gln Ala Pro Gly Lys Glu Arg Glu Gly Val
        35                  40                  45


Ser Cys Thr Ser Ser Ser Asp Gly Ser Thr Asn Tyr Ser Asp Ser Val
    50                  55                  60


Lys Gly Arg Phe Ile Ile Ser Arg Asp Asn Ala Lys Asn Thr Val Tyr
65                  70                  75                  80


Leu Gln Met Asn Ser Leu Lys Pro Glu Asp Thr Ala Val Tyr Tyr Cys
                85                  90                  95


Ala Val Thr Pro Ala Thr Tyr Tyr Gly Gly Arg Tyr Tyr Phe Gln Cys
            100                 105                 110


Pro Tyr His Asp Met His Tyr Trp Gly Lys Gly Thr Leu Val Thr Val

        115                 120                 125

Ser Ser
    130

<210>  22
<211>  9
<212>  PRT
<213>  Artificial Sequence

<220>
<223>  Re31A12 CDR1

<400>  22

Val Thr Leu Asp Tyr Tyr Ala Ile Gly
1               5

<210>  23
<211>  15
<212>  PRT
<213>  Artificial Sequence

<220>
<223>  Re31A12 CDR2

<400>  23

Ser Cys Thr Ser Ser Ser Asp Gly Ser Thr Asn Tyr Ser Asp Ser
1               5                   10                  15

<210>  24
<211>  22
<212>  PRT
<213>  Artificial Sequence

<220>
<223>  Re31A12 CDR3

<400>  24

Val Thr Pro Ala Thr Tyr Tyr Gly Gly Arg Tyr Tyr Phe Gln Cys Pro
1               5                   10                  15

Tyr His Asp Met His Tyr
            20

<210>  25
<211>  130
<212>  PRT
<213>  Artificial Sequence

<220>
<223>  Ma02H07

<400>  25

Gln Val Gln Leu Val Glu Ser Gly Gly Gly Leu Val Gln Pro Gly Gly
1               5                   10                  15

Ser Leu Arg Leu Ser Cys Ala Tyr Ser Gly Ile Thr Leu Asp Tyr Tyr
            20                  25                  30

Ala Ile Gly Trp Phe Arg Gln Ala Pro Gly Lys Glu Arg Glu Gly Val
        35                  40                  45

Ser Tyr Ile Ser Ser Ser Asn Asp Arg Thr Asp Tyr Ala Asp Ser Val
    50                  55                  60

Lys Gly Arg Phe Thr Ile Ser Arg Asp Asn Ala Lys Asn Thr Val Phe
65                  70                  75                  80

Leu Gln Met Asn Ser Leu Gln Pro Glu Asp Thr Ala Val Tyr Tyr Cys
                85                  90                  95

Ala Thr Val Thr Gly Thr Tyr Tyr Ser Gly Arg Trp Tyr Tyr Asn Trp
            100                 105                 110

His Pro Glu Ala Val Asp Tyr Trp Gly Lys Gly Thr Leu Val Thr Val
        115                 120                 125

Ser Ser
    130

<210>  26
<211>  9
<212>  PRT
<213>  Artificial Sequence

<220>
<223>  Ma02H07 CDR1

<400>  26

Ile Thr Leu Asp Tyr Tyr Ala Ile Gly
1               5

<210>  27
<211>  15
<212>  PRT
<213>  Artificial Sequence

<220>
<223>  Ma02H07 CDR2

<400>  27

Ser Tyr Ile Ser Ser Ser Asn Asp Arg Thr Asp Tyr Ala Asp Ser
1               5                   10                  15

<210>  28
<211>  22
<212>  PRT
<213>  Artificial Sequence

<220>
<223>  Ma02H07 CDR3

<400>  28

Thr Val Thr Gly Thr Tyr Tyr Ser Gly Arg Trp Tyr Tyr Asn Trp His
1               5                   10                  15

Pro Glu Ala Val Asp Tyr
            20

<210>  29
<211>  130
<212>  PRT
<213>  Artificial Sequence

<220>
<223>  Ma02F10

<400>  29

Gln Val Gln Leu Val Glu Ser Gly Gly Gly Leu Val Gln Pro Gly Gly
1               5                   10                  15

Ser Leu Arg Leu Ser Cys Ala Tyr Ser Gly Ile Thr Leu Asp Tyr Tyr
            20                  25                  30

Ala Ile Gly Trp Phe Arg Gln Ala Pro Gly Lys Glu Arg Glu Gly Val
        35                  40                  45

Ser Tyr Ile Ser Gly Ser Asn Asp Arg Thr Asp Tyr Ala Asp Ser Val
    50                  55                  60

Lys Gly Arg Phe Thr Ile Ser Arg Asp Asn Ala Lys Asn Thr Val Phe
65                  70                  75                  80

Leu Gln Met Asn Ser Leu Glu Pro Glu Asp Thr Ala Val Tyr Tyr Cys
                85                  90                  95

Ala Thr Val Thr Gly Thr Tyr Tyr Ser Gly Arg Trp Tyr Tyr Asn Trp
            100                 105                 110

His Pro Glu Ala Val Asp Tyr Trp Gly Lys Gly Thr Leu Val Thr Val
        115                 120                 125

Ser Ser
    130

<210>  30
<211>  9
<212>  PRT
<213>  Artificial Sequence

<220>
<223>  Ma02F10 CDR1

<400>  30

Ile Thr Leu Asp Tyr Tyr Ala Ile Gly
1               5


<210>  31
<211>  15
<212>  PRT
<213>  Artificial Sequence

<220>
<223>  Ma02F10 CDR2

<400>  31

Ser Tyr Ile Ser Gly Ser Asn Asp Arg Thr Asp Tyr Ala Asp Ser
1               5                   10                  15


<210>  32
<211>  22
<212>  PRT
<213>  Artificial Sequence

<220>
<223>  Ma02F10 CDR3

<400>  32

Thr Val Thr Gly Thr Tyr Tyr Ser Gly Arg Trp Tyr Tyr Asn Trp His
1               5                   10                  15


Pro Glu Ala Val Asp Tyr
            20


<210>  33
<211>  130
<212>  PRT
<213>  Artificial Sequence

<220>
<223>  Ma02E06

<400>  33

Gln Val Gln Leu Val Glu Ser Gly Gly Asp Leu Val Gln Pro Arg Gly
1               5                   10                  15


Ser Leu Arg Leu Ser Cys Ala Ala Ser Gly Ile Thr Leu Asp Tyr Tyr

20                  25                  30

Ala Ile Gly Trp Phe Arg Gln Ala Pro Gly Lys Glu Arg Glu Gly Val
        35                  40                  45

Ser Tyr Ile Ser Asn Thr Asn Asp Lys Thr Asp Tyr Ala Asp Ser Val
    50                  55                  60

Lys Gly Arg Phe Thr Ile Ser Arg Asp Asn Ala Glu Asn Thr Val Phe
65                  70                  75                  80

Leu Gln Met Asn Ser Leu Gln Pro Glu Asp Thr Ala Val Tyr Tyr Cys
                85                  90                  95

Ala Thr Val Pro Gly Thr Tyr Tyr Gly Gly Arg Trp Tyr Tyr Thr Trp
            100                 105                 110

His Pro Glu Ala Val Arg Tyr Trp Gly Lys Gly Thr Gln Val Thr Val
        115                 120                 125

Ser Ser
    130

<210>  34
<211>  9
<212>  PRT
<213>  Artificial Sequence

<220>
<223>  Ma02E06 CDR1

<400>  34

Ile Thr Leu Asp Tyr Tyr Ala Ile Gly
1               5

<210>  35
<211>  15
<212>  PRT
<213>  Artificial Sequence

<220>
<223>  Ma02E06 CDR2

<400>  35

Ser Tyr Ile Ser Asn Thr Asn Asp Lys Thr Asp Tyr Ala Asp Ser
1               5                   10                  15

<210>  36
<211>  22
<212>  PRT
<213>  Artificial Sequence

<220>
<223>  Ma02E06 CDR3

<400>  36

Thr Val Pro Gly Thr Tyr Tyr Gly Gly Arg Trp Tyr Tyr Thr Trp His
1               5                   10                  15

Pro Glu Ala Val Arg Tyr
            20

<210>  37
<211>  128
<212>  PRT
<213>  Artificial Sequence

<220>
<223>  Re32D03

<400>  37

Gln Val Gln Leu Val Glu Ser Gly Gly Gly Leu Val Gln Pro Gly Gly
1               5                   10                  15

Ser Leu Arg Leu Ser Cys Ala Ile Ser Gly Ile Thr Leu Asp Tyr Tyr
            20                  25                  30

Ala Val Gly Trp Phe Leu Gln Ala Pro Gly Lys Glu Arg Glu Gly Ile
        35                  40                  45

Ser Cys Met Arg Asn Trp Asp Gly Arg Thr Val Tyr Ala Pro Ser Val
    50                  55                  60

Lys Gly Arg Phe Thr Ile Ser Ser Asp Asn Ala Lys Lys Met Val Tyr
65                  70                  75                  80

Leu Glu Met Asp Asn Leu Lys Ser Glu Asp Thr Gly Val Tyr Tyr Cys
                85                  90                  95

Ala Ala Gly Pro Leu Pro Pro Gly Ile Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210>  38
<211>  9
<212>  PRT
<213>  Artificial Sequence

<220>
<223>  Re32D03 CDR1

<400>  38

Ile Thr Leu Asp Tyr Tyr Ala Val Gly
1               5


<210>  39
<211>  15
<212>  PRT
<213>  Artificial Sequence

<220>
<223>  Re32D03 CDR2

<400>  39

Ser Cys Met Arg Asn Trp Asp Gly Arg Thr Val Tyr Ala Pro Ser
1               5                   10                  15


<210>  40
<211>  20
<212>  PRT
<213>  Artificial Sequence

<220>
<223>  Re32D03 CDR3

<400>  40

Ala Gly Pro Leu Pro Pro Gly Ile Ser Cys Arg Ile Pro Thr Pro Leu
1               5                   10                  15


Gly Tyr Asp Asp
            20


<210>  41
<211>  128
<212>  PRT
<213>  Artificial Sequence

<220>
<223>  Re32G06

<400>  41

Gln Val Gln Leu Val Glu Ser Gly Gly Gly Leu Val Gln Pro Gly Gly
1               5                   10                  15


Ser Leu Arg Leu Ser Cys Ala Ile Ser Gly Ile Thr Leu Asp Tyr Tyr
            20                  25                  30


Ala Val Gly Trp Phe Leu Gln Ala Pro Gly Lys Glu Arg Glu Gly Ile
        35                  40                  45


Ser Cys Met Arg Asn Trp Asp Gly Arg Thr Val Tyr Ala Pro Ser Val
    50                  55                  60

Lys Gly Arg Phe Thr Ile Ser Ser Asp Asn Ala Lys Lys Met Val Tyr
65                  70                  75                  80

Leu Glu Met Asn Ser Leu Lys Ser Glu Asp Thr Gly Val Tyr Tyr Cys
                85                  90                  95

Ala Ala Gly Pro Leu Pro Pro Gly Thr Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210>  42
<211>  9
<212>  PRT
<213>  Artificial Sequence

<220>
<223>  Re32G06 CDR1

<400>  42

Ile Thr Leu Asp Tyr Tyr Ala Val Gly
1               5

<210>  43
<211>  15
<212>  PRT
<213>  Artificial Sequence

<220>
<223>  Re32G06 CDR2

<400>  43

Ser Cys Met Arg Asn Trp Asp Gly Arg Thr Val Tyr Ala Pro Ser
1               5                   10                  15

<210>  44
<211>  20
<212>  PRT
<213>  Artificial Sequence

<220>
<223>  Re32G06 CDR3

<400>  44

Ala Gly Pro Leu Pro Pro Gly Thr Ser Cys Arg Ile Pro Thr Pro Leu
1               5                   10                  15

Gly Tyr Asp Asp
            20

<210>  45
<211>  128
<212>  PRT
<213>  Artificial Sequence

<220>
<223>  Re32B11

<400>  45

Gln Val Gln Leu Val Glu Ser Gly Gly Gly Leu Val Gln Pro Gly Gly
1               5                   10                  15

Ser Leu Arg Leu Ser Cys Ala Thr Ser Gly Ile Thr Leu Asp Tyr Tyr
            20                  25                  30

Ala Ile Gly Trp Phe Leu Gln Ala Pro Gly Lys Glu Arg Glu Gly Val
        35                  40                  45

Ser Cys Met Arg Asn Ser Asp Gly Arg Thr Val Tyr Ala Pro Ser Val
    50                  55                  60

Lys Gly Arg Phe Thr Ile Ser Arg Asp Asn Ala Lys Lys Met Val Tyr
65                  70                  75                  80

Leu Glu Met Asn Asn Leu Lys Ser Glu Asp Thr Gly Val Tyr Tyr Cys
                85                  90                  95

Ala Ala Gly Pro Leu Pro Pro Gly Ile Ser Cys Arg Ile Pro Thr Pro
            100                 105                 110

Leu Gly Tyr Asp Asp Trp Gly Gln Gly Thr Gln Val Thr Val Ser Ser
        115                 120                 125


<210>  46
<211>  9
<212>  PRT
<213>  Artificial Sequence

<220>
<223>  Re32B11 CDR1

<400>  46

Ile Thr Leu Asp Tyr Tyr Ala Ile Gly
1               5


<210>  47
<211>  15
<212>  PRT
<213>  Artificial Sequence

<220>

<223>  Re32B11 CDR2

<400>  47

Ser Cys Met Arg Asn Ser Asp Gly Arg Thr Val Tyr Ala Pro Ser
1               5                   10                  15


<210>  48
<211>  20
<212>  PRT
<213>  Artificial Sequence

<220>
<223>  Re32B11 CDR3

<400>  48

Ala Gly Pro Leu Pro Pro Gly Ile Ser Cys Arg Ile Pro Thr Pro Leu
1               5                   10                  15


Gly Tyr Asp Asp
            20


<210>  49
<211>  128
<212>  PRT
<213>  Artificial Sequence

<220>
<223>  Re32A10

<400>  49

Gln Val Gln Leu Val Glu Ser Gly Gly Gly Leu Val Gln Pro Gly Gly
1               5                   10                  15


Ser Leu Arg Leu Ser Cys Ala Thr Ser Gly Ile Thr Leu Asp Tyr Tyr
            20                  25                  30


Ala Ile Gly Trp Phe Leu Gln Ala Pro Gly Lys Glu Arg Glu Gly Val
        35                  40                  45


Ser Cys Met Arg Ser Ser Asp Gly Ser Thr Val Tyr Ala Pro Ser Val
    50                  55                  60


Lys Gly Arg Phe Thr Ile Ser Arg Asp Asn Ala Lys Lys Met Val Tyr
65                  70                  75                  80


Leu Glu Met Asn Ser Leu Lys Ser Glu Asp Thr Gly Val Tyr Tyr Cys
                85                  90                  95


Ala Ala Gly Pro Leu Pro Pro Gly Thr Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210>  50
<211>  9
<212>  PRT
<213>  Artificial Sequence

<220>
<223>  Re32A10 CDR1

<400>  50

Ile Thr Leu Asp Tyr Tyr Ala Ile Gly
1               5

<210>  51
<211>  15
<212>  PRT
<213>  Artificial Sequence

<220>
<223>  Re32A10 CDR2

<400>  51

Ser Cys Met Arg Ser Ser Asp Gly Ser Thr Val Tyr Ala Pro Ser
1               5                   10                  15

<210>  52
<211>  20
<212>  PRT
<213>  Artificial Sequence

<220>
<223>  Re32A10 CDR3

<400>  52

Ala Gly Pro Leu Pro Pro Gly Thr Ser Cys Arg Ile Pro Thr Pro Leu
1               5                   10                  15

Gly Tyr Asp Asp
            20

<210>  53
<211>  128
<212>  PRT
<213>  Artificial Sequence

<220>
<223>  Re32B02

<400>  53

Gln Val Gln Leu Val Glu Ser Gly Gly Gly Leu Val Gln Pro Gly Gly
1               5                   10                  15

Ser Leu Arg Leu Ser Cys Ala Thr Ser Gly Ile Thr Leu Asp Tyr Tyr
            20                  25                  30

Ala Ile Gly Trp Phe Leu Gln Ala Pro Gly Lys Glu Arg Glu Gly Val
        35                  40                  45

Ser Cys Met Arg Ser Ser Asp Gly Ser Thr Val Tyr Ala Pro Ser Val
    50                  55                  60

Lys Gly Arg Phe Thr Ile Ser Arg Asp Asn Ala Lys Lys Met Val Tyr
65                  70                  75                  80

Leu Glu Met Asn Ser Leu Lys Ser Glu Asp Thr Gly Val Tyr Tyr Cys
                85                  90                  95

Ala Ala Gly Pro Leu Pro Pro Gly Thr Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210> 54
<211> 9
<212> PRT
<213> Artificial Sequence

<220>
<223> Re32B02 CDR1

<400> 54

Ile Thr Leu Asp Tyr Tyr Ala Ile Gly
1               5

<210> 55
<211> 15
<212> PRT
<213> Artificial Sequence

<220>
<223> Re32B02 CDR2

<400> 55

Ser Cys Met Arg Ser Ser Asp Gly Ser Thr Val Tyr Ala Pro Ser
1               5                   10                  15

<210> 56
<211> 20
<212> PRT
<213> Artificial Sequence

<220>
<223>  Re32B02 CDR3

<400>  56

Ala Gly Pro Leu Pro Pro Gly Thr Ser Cys Arg Ile Pro Thr Pro Leu
1               5                   10                  15

Gly Tyr Asp Asp
            20

<210>  57
<211>  128
<212>  PRT
<213>  Artificial Sequence

<220>
<223>  Re32F03

<400>  57

Gln Val Gln Leu Val Glu Ser Gly Gly Gly Leu Val Gln Pro Gly Gly
1               5                   10                  15

Ser Leu Arg Leu Ser Cys Ala Thr Ser Gly Ile Thr Leu Asp Tyr Tyr
            20                  25                  30

Ala Ile Gly Trp Phe Leu Gln Ala Pro Gly Lys Glu Arg Glu Gly Val
        35                  40                  45

Ala Cys Met Arg Ser Ser Asp Glu Arg Ser Leu Tyr Ala Pro Ser Val
    50                  55                  60

Lys Gly Arg Phe Thr Ile Ser Arg Asp Asn Ala Lys Lys Thr Val Tyr
65                  70                  75                  80

Leu Glu Met Asn Ser Leu Lys Pro Glu Asp Thr Gly Val Tyr Tyr Cys
                85                  90                  95

Ala Ser Gly Ser Val Val Pro Gly Thr Met Cys Arg Ile Pro Thr Pro
            100                 105                 110

Leu Gly Tyr Asp Asp Trp Gly Gln Gly Thr Gln Val Thr Val Ser Ser
        115                 120                 125

<210>  58
<211>  9
<212>  PRT
<213>  Artificial Sequence

<220>
<223>  Re32F03 CDR1

<400>  58

Ile Thr Leu Asp Tyr Tyr Ala Ile Gly
1               5


<210>  59
<211>  15
<212>  PRT
<213>  Artificial Sequence

<220>
<223>  Re32F03 CDR2

<400>  59

Ala Cys Met Arg Ser Ser Asp Glu Arg Ser Leu Tyr Ala Pro Ser
1               5                   10                  15


<210>  60
<211>  20
<212>  PRT
<213>  Artificial Sequence

<220>
<223>  Re32F03 CDR3

<400>  60

Ser Gly Ser Val Val Pro Gly Thr Met Cys Arg Ile Pro Thr Pro Leu
1               5                   10                  15


Gly Tyr Asp Asp
            20


<210>  61
<211>  128
<212>  PRT
<213>  Artificial Sequence

<220>
<223>  Re32C08

<400>  61

Gln Val Gln Leu Val Glu Ser Gly Gly Gly Leu Val Gln Pro Gly Gly
1               5                   10                  15


Ser Leu Arg Leu Ser Cys Glu Thr Ser Gly Ile Thr Leu Asp Tyr Tyr
            20                  25                  30


Ala Ile Gly Trp Phe Leu Gln Ala Pro Gly Lys Glu Arg Glu Gly Val
        35                  40                  45


Ala Cys Met Arg Asn Trp Asp Gly Ser Thr Val Tyr Ala Pro Ser Val
    50                  55                  60

Lys Gly Arg Phe Thr Ile Ser Arg Asp Asn Asp Lys Lys Ala Ala Tyr
65                  70                  75                  80

Leu Glu Met Asn Ser Leu Lys Ser Glu Asp Thr Gly Val Tyr Tyr Cys
                85                  90                  95

Ala Ala Gly Pro Leu Pro Pro Gly His Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210>  62
<211>  9
<212>  PRT
<213>  Artificial Sequence

<220>
<223>  Re32C08 CDR1

<400>  62

Ile Thr Leu Asp Tyr Tyr Ala Ile Gly
1               5

<210>  63
<211>  15
<212>  PRT
<213>  Artificial Sequence

<220>
<223>  Re32C08 CDR2

<400>  63

Ala Cys Met Arg Asn Trp Asp Gly Ser Thr Val Tyr Ala Pro Ser
1               5                   10                  15

<210>  64
<211>  20
<212>  PRT
<213>  Artificial Sequence

<220>
<223>  Re32C08 CDR3

<400>  64

Ala Gly Pro Leu Pro Pro Gly His Ser Cys Arg Ile Pro Thr Pro Leu
1               5                   10                  15

Gly Tyr Asp Asp
            20

<210>  65
<211>  128
<212>  PRT
<213>  Artificial Sequence

<220>
<223>  Re32G01

<400>  65

Gln Val Gln Leu Val Glu Ser Gly Gly Gly Leu Val Gln Pro Gly Gly
1               5                   10                  15

Ser Leu Arg Leu Ser Cys Glu Thr Ser Gly Ile Thr Leu Asp Tyr Tyr
            20                  25                  30

Ala Ile Gly Trp Phe Leu Gln Ala Pro Gly Lys Asp Arg Glu Gly Val
        35                  40                  45

Ala Cys Met Arg Asn Trp Asp Gly Ser Thr Leu Tyr Ala Pro Ser Val
    50                  55                  60

Lys Gly Arg Phe Thr Ile Ser Arg Asp Ala Asp Lys Glu Val Ala Tyr
65                  70                  75                  80

Leu Glu Met Asn Ser Leu Lys Ser Glu Asp Thr Gly Val Tyr Tyr Cys
                85                  90                  95

Ala Ala Gly Pro Leu Pro Pro Gly His Ser Cys Arg Ile Pro Thr Pro
            100                 105                 110

Leu Gly Tyr Asp Asp Trp Gly Gln Gly Thr Gln Val Thr Val Ser Ser
        115                 120                 125


<210>  66
<211>  9
<212>  PRT
<213>  Artificial Sequence

<220>
<223>  Re32G01 CDR1

<400>  66

Ile Thr Leu Asp Tyr Tyr Ala Ile Gly
1               5


<210>  67
<211>  15
<212>  PRT
<213>  Artificial Sequence

<220>
<223>  Re32G01 CDR2

<400>  67

Ala Cys Met Arg Asn Trp Asp Gly Ser Thr Leu Tyr Ala Pro Ser
1               5                   10                  15


<210>  68
<211>  20
<212>  PRT
<213>  Artificial Sequence

<220>
<223>  Re32G01 CDR3

<400>  68

Ala Gly Pro Leu Pro Pro Gly His Ser Cys Arg Ile Pro Thr Pro Leu
1               5                   10                  15


Gly Tyr Asp Asp
            20


<210>  69
<211>  128
<212>  PRT
<213>  Artificial Sequence

<220>
<223>  Re32F02

<400>  69

Gln Val Gln Leu Val Glu Ser Gly Gly Gly Leu Val Ser Pro Gly Gly
1               5                   10                  15


Ser Leu Lys Leu Ser Cys Ala Ala Ser Gly Leu Thr Leu Asp Tyr Tyr
            20                  25                  30


Gly Ile Gly Trp Phe Arg Gln Val Pro Gly Lys Glu Arg Glu Gly Val
        35                  40                  45


Ser Cys Leu Gly Val Pro Asp Asn Arg Thr Leu Tyr Ala Pro Ser Val
    50                  55                  60


Lys Gly Arg Phe Thr Ile Ser Arg Asp Asn Ala Lys Asn Thr Tyr Tyr
65                  70                  75                  80


Leu Glu Met Asn Ser Leu Asn Pro Glu Asp Ser Ala Val Tyr Tyr Cys
                85                  90                  95


Ala Ala Gly Pro Val Val Pro Gly His Met Cys Arg Ile Pro Thr Pro
            100                 105                 110

Leu Gly Tyr Asp Tyr Trp Gly Gln Gly Thr Leu Val Thr Val Ser Ser
        115                 120                 125

<210>  70
<211>  9
<212>  PRT
<213>  Artificial Sequence

<220>
<223>  Re32F02 CDR1

<400>  70

Leu Thr Leu Asp Tyr Tyr Gly Ile Gly
1               5

<210>  71
<211>  15
<212>  PRT
<213>  Artificial Sequence

<220>
<223>  Re32F02 CDR2

<400>  71

Ser Cys Leu Gly Val Pro Asp Asn Arg Thr Leu Tyr Ala Pro Ser
1               5                   10                  15

<210>  72
<211>  20
<212>  PRT
<213>  Artificial Sequence

<220>
<223>  Re32F02 CDR3

<400>  72

Ala Gly Pro Val Val Pro Gly His Met Cys Arg Ile Pro Thr Pro Leu
1               5                   10                  15

Gly Tyr Asp Tyr
            20

<210>  73
<211>  128
<212>  PRT
<213>  Artificial Sequence

<220>
<223>  Re32D08

<400>  73

Gln Val Gln Leu Val Glu Ser Gly Gly Gly Leu Val Ser Pro Gly Gly
1               5                   10                  15

Ser Leu Lys Leu Ser Cys Ala Ala Ser Gly Leu Thr Leu Asp Tyr Tyr
            20                  25                  30

Gly Ile Gly Trp Phe Arg Gln Val Pro Gly Lys Glu Arg Glu Gly Val
        35                  40                  45

Ser Cys Leu Gly Val Pro Asp Asn Arg Thr Leu Tyr Ala Pro Ser Val
    50                  55                  60

Lys Gly Arg Phe Thr Ile Ser Arg Asp Asn Ala Lys Asn Thr Phe Tyr
65                  70                  75                  80

Leu Glu Met Asn Ser Leu Asn Pro Glu Asp Ser Ala Val Tyr Tyr Cys
                85                  90                  95

Ala Ala Gly Pro Val Val Pro Gly His Met Cys Arg Ile Pro Thr Pro
            100                 105                 110

Leu Gly Tyr Asp Tyr Trp Gly Gln Gly Thr Gln Val Thr Val Ser Ser
        115                 120                 125

<210>  74
<211>  9
<212>  PRT
<213>  Artificial Sequence

<220>
<223>  Re32D08 CDR1

<400>  74

Leu Thr Leu Asp Tyr Tyr Gly Ile Gly
1               5

<210>  75
<211>  15
<212>  PRT
<213>  Artificial Sequence

<220>
<223>  Re32D08 CDR2

<400>  75

Ser Cys Leu Gly Val Pro Asp Asn Arg Thr Leu Tyr Ala Pro Ser
1               5                   10                  15

<210>  76
<211>  20
<212>  PRT
<213>  Artificial Sequence

<220>

<223>  Re32D08 CDR3

<400>  76

Ala Gly Pro Val Val Pro Gly His Met Cys Arg Ile Pro Thr Pro Leu
1               5                   10                  15

Gly Tyr Asp Tyr
            20

<210>  77
<211>  130
<212>  PRT
<213>  Artificial Sequence

<220>
<223>  Re30H02

<400>  77

Gln Val Gln Leu Val Glu Ser Gly Gly Asp Ser Val Gln Pro Gly Asp
1               5                   10                  15

Ser Leu Arg Leu Ser Cys Ala Ile Ser Gly Phe Thr Leu Asp Tyr Tyr
            20                  25                  30

Thr Ile Gly Trp Phe Arg Gln Ala Pro Gly Lys Glu Arg Glu Gly Val
        35                  40                  45

Ser Cys Ile Gly Ser Ser Asp Ser Ser Thr Ser Tyr Ala Asp Ala Val
    50                  55                  60

Lys Gly Arg Phe Thr Ile Ser Ser Asp Asn Ala Lys Asn Thr Val Tyr
65                  70                  75                  80

Leu Gln Met Asn Asn Leu Lys Pro Glu Asp Thr Ala Val Tyr Tyr Cys
                85                  90                  95

Ala Ala Gly Pro Ala Thr Tyr Tyr Gly Gly Asn Trp His His Thr Cys
            100                 105                 110

His Ala Asn Ala Met His Tyr Trp Gly Lys Gly Thr Leu Val Thr Val
        115                 120                 125

Ser Ser
    130

<210>  78
<211>  9
<212>  PRT
<213>  Artificial Sequence

<220>
<223>  Re30H02 CDR1

<400>  78

Phe Thr Leu Asp Tyr Tyr Thr Ile Gly
1               5

<210>  79
<211>  15
<212>  PRT
<213>  Artificial Sequence

<220>
<223>  Re30H02 CDR2

<400>  79

Ser Cys Ile Gly Ser Ser Asp Ser Ser Thr Ser Tyr Ala Asp Ala
1               5                   10                  15

<210>  80
<211>  22
<212>  PRT
<213>  Artificial Sequence

<220>
<223>  Re30H02 CDR3

<400>  80

Ala Gly Pro Ala Thr Tyr Tyr Gly Gly Asn Trp His His Thr Cys His
1               5                   10                  15

Ala Asn Ala Met His Tyr
            20

<210>  81
<211>  130
<212>  PRT
<213>  Artificial Sequence

<220>
<223>  Re30B09

<400>  81

Gln Val Gln Leu Val Glu Ser Gly Gly Gly Leu Val Gln Pro Gly Gly
1               5                   10                  15

Ser Leu Arg Leu Ser Cys Glu Ile Ser Gly Phe Thr Leu Asp Tyr Tyr
            20                  25                  30

Thr Ile Gly Trp Phe Arg Gln Ala Pro Gly Lys Glu Arg Glu Gly Val
        35                  40                  45

Ser Cys Ile Gly Ser Ser Asp Ser Ser Thr Ser Tyr Ala Asp Ala Val
    50                  55                  60

Lys Gly Arg Phe Thr Ile Ser Ser Asp Asn Ala Lys Asn Thr Val Tyr
65                  70                  75                  80

Leu Gln Met Asn Asn Leu Lys Pro Glu Asp Thr Ala Val Tyr Tyr Cys
                85                  90                  95

Ala Ala Gly Pro Ala Thr Tyr Tyr Gly Gly Asn Trp His His Thr Cys
            100                 105                 110

His Ala Asn Ala Met Asp Tyr Trp Gly Lys Gly Thr Leu Val Thr Val
        115                 120                 125

Ser Ser
    130

<210>  82
<211>  9
<212>  PRT
<213>  Artificial Sequence

<220>
<223>  Re30B09 CDR1

<400>  82

Phe Thr Leu Asp Tyr Tyr Thr Ile Gly
1               5

<210>  83
<211>  15
<212>  PRT
<213>  Artificial Sequence

<220>
<223>  Re30B09 CDR2

<400>  83

Ser Cys Ile Gly Ser Ser Asp Ser Ser Thr Ser Tyr Ala Asp Ala
1               5                   10                  15

<210>  84
<211>  22
<212>  PRT
<213>  Artificial Sequence

<220>
<223>  Re30B09 CDR3

<400>  84

Ala Gly Pro Ala Thr Tyr Tyr Gly Gly Asn Trp His His Thr Cys His

1               5                   10                  15

Ala Asn Ala Met Asp Tyr
            20

<210>  85
<211>  130
<212>  PRT
<213>  Artificial Sequence

<220>
<223>  Re30A12

<400>  85

Gln Val Gln Leu Val Glu Ser Gly Gly Gly Leu Val Gln Pro Gly Gly
1               5                   10                  15

Ser Leu Arg Leu Ser Cys Ala Ala Ser Gly Val Thr Leu Asp Tyr Tyr
            20                  25                  30

Thr Ile Gly Trp Phe Arg Gln Ala Pro Gly Lys Glu Arg Glu Gly Val
        35                  40                  45

Ser Cys Ile Gly Ser Gly Asp Gly Ser Thr Ser Tyr Ala Asp Ala Val
    50                  55                  60

Lys Gly Arg Phe Thr Ile Ser Asn Asp Asn Ala Lys Asn Thr Val Tyr
65                  70                  75                  80

Leu Gln Met Asn Asn Leu Lys Pro Glu Asp Thr Ala Val Tyr Tyr Cys
                85                  90                  95

Ala Val Gly Pro Ala Thr Tyr Tyr Gly Gly Asn Trp His Tyr Thr Cys
            100                 105                 110

His Ala Asp Ala Met Arg Asp Trp Gly Lys Gly Thr Leu Val Thr Val
        115                 120                 125

Ser Ser
    130

<210>  86
<211>  9
<212>  PRT
<213>  Artificial Sequence

<220>
<223>  Re30A12 CDR1

<400>  86

Val Thr Leu Asp Tyr Tyr Thr Ile Gly
1               5

<210>  87
<211>  15
<212>  PRT
<213>  Artificial Sequence

<220>
<223>  Re30A12 CDR2

<400>  87

Ser Cys Ile Gly Ser Gly Asp Gly Ser Thr Ser Tyr Ala Asp Ala
1               5                   10                  15

<210>  88
<211>  22
<212>  PRT
<213>  Artificial Sequence

<220>
<223>  Re30A12 CDR3

<400>  88

Val Gly Pro Ala Thr Tyr Tyr Gly Gly Asn Trp His Tyr Thr Cys His
1               5                   10                  15

Ala Asp Ala Met Arg Asp
            20

<210>  89
<211>  123
<212>  PRT
<213>  Artificial Sequence

<220>
<223>  Re30D01

<400>  89

Gln Val Gln Leu Val Glu Ser Gly Gly Gly Leu Val Gln Ala Gly Gly
1               5                   10                  15

Ser Leu Arg Leu Ser Cys Ala Ala Ser Gly Pro Ile Phe Ser Phe Asn
            20                  25                  30

Ala Val Asp Trp Tyr Arg Gln Ala Pro Gly Lys Gln Arg Glu Leu Val
        35                  40                  45

Ala Gln Ile Gly His His Gly Ala Thr Asn Tyr Val Asp Ser Val Lys
    50                  55                  60

Gly Arg Phe Thr Ile Ser Lys Asp Ser Ala Lys Asn Thr Ala Tyr Leu

65                  70                  75                  80

Gln Met Asp Asn Leu Lys Pro Glu Asp Thr Ala Val Tyr Arg Cys Asn
                85                  90                  95

Ala Phe Val Ser Ile Asn Ile Pro Gln Gly Arg Glu Glu Phe Gly Tyr
            100                 105                 110

Trp Gly Gln Gly Thr Gln Val Thr Val Ser Ser
        115                 120

<210>  90
<211>  9
<212>  PRT
<213>  Artificial Sequence

<220>
<223>  Re30D01 CDR1

<400>  90

Pro Ile Phe Ser Phe Asn Ala Val Asp
1               5

<210>  91
<211>  14
<212>  PRT
<213>  Artificial Sequence

<220>
<223>  Re30D01 CDR2

<400>  91

Ala Gln Ile Gly His His Gly Ala Thr Asn Tyr Val Asp Ser
1               5                   10

<210>  92
<211>  16
<212>  PRT
<213>  Artificial Sequence

<220>
<223>  Re30D01 CDR3

<400>  92

Ala Phe Val Ser Ile Asn Ile Pro Gln Gly Arg Glu Glu Phe Gly Tyr
1               5                   10                  15

<210>  93
<211>  132
<212>  PRT
<213>  Artificial Sequence

<220>

<223>  Re30A03

<400>  93

Gln Val Gln Leu Val Glu Ser Gly Gly Gly Leu Val Gln Pro Gly Asp
1               5                   10                  15

Ser Leu Thr Leu Ser Cys Ala Thr Thr Glu Val Thr Leu Asp Tyr Tyr
            20                  25                  30

Gly Leu Cys Trp Phe Arg Gln Ala Pro Gly Lys Gly Arg Glu Gly Val
        35                  40                  45

Ala Cys Ile Ser Ser Val Asp Gly Ala Thr Val Tyr Ala Asp Ser Val
    50                  55                  60

Lys Gly Arg Phe Thr Ile Ser Arg Asp Asn Ser Lys Asn Thr Val Ser
65                  70                  75                  80

Leu Gln Met Asn Asp Leu Ser Leu Glu Asp Thr Ala Val Tyr Ser Cys
                85                  90                  95

Gly Ala Gly Pro Met Thr Pro Tyr Gly Gly Arg Trp Tyr Phe Ser Gly
            100                 105                 110

Leu Leu Ala Gly Asn Gly Met His Tyr Trp Gly Lys Gly Thr Leu Val
        115                 120                 125

Thr Val Ser Ser
    130


<210>  94
<211>  9
<212>  PRT
<213>  Artificial Sequence

<220>
<223>  Re30A03 CDR1

<400>  94

Val Thr Leu Asp Tyr Tyr Gly Leu Cys
1               5


<210>  95
<211>  15
<212>  PRT
<213>  Artificial Sequence

<220>
<223>  Re30A03 CDR2

<400>  95

Ala Cys Ile Ser Ser Val Asp Gly Ala Thr Val Tyr Ala Asp Ser
1               5                   10                  15

<210>  96
<211>  24
<212>  PRT
<213>  Artificial Sequence

<220>
<223>  Re30A03 CDR3

<400>  96

Ala Gly Pro Met Thr Pro Tyr Gly Gly Arg Trp Tyr Phe Ser Gly Leu
1               5                   10                  15

Leu Ala Gly Asn Gly Met His Tyr
            20

<210>  97
<211>  130
<212>  PRT
<213>  Artificial Sequence

<220>
<223>  Re30E02

<400>  97

Gln Val Gln Leu Val Glu Ser Gly Gly Gly Leu Val Gln Pro Gly Gly
1               5                   10                  15

Ser Leu Arg Leu Ser Cys Ala Ala Ser Gly Leu Thr Leu Asp Tyr Tyr
            20                  25                  30

Thr Ile Gly Trp Phe Arg Gln Ala Pro Gly Lys Glu Arg Glu Gly Val
        35                  40                  45

Thr Cys Ile Ala Ser Ser Asp Gly Asn Thr Val Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Gly Gly Pro Gln Thr Tyr Tyr Gly Gly Thr Tyr His Ser Thr Cys
            100                 105                 110

His Glu Asn Ala Met Asp Tyr Trp Gly Lys Gly Thr Leu Val Thr Val
        115                 120                 125

Ser Ser
    130


<210>  98
<211>  9
<212>  PRT
<213>  Artificial Sequence

<220>
<223>  Re30E02 CDR1

<400>  98

Leu Thr Leu Asp Tyr Tyr Thr Ile Gly
1               5


<210>  99
<211>  15
<212>  PRT
<213>  Artificial Sequence

<220>
<223>  Re30E02 CDR2

<400>  99

Thr Cys Ile Ala Ser Ser Asp Gly Asn Thr Val Tyr Ala Asp Ser
1               5                   10                  15


<210>  100
<211>  22
<212>  PRT
<213>  Artificial Sequence

<220>
<223>  Re30E02 CDR3

<400>  100

Gly Gly Pro Gln Thr Tyr Tyr Gly Gly Thr Tyr His Ser Thr Cys His
1               5                   10                  15


Glu Asn Ala Met Asp Tyr
            20


<210>  101
<211>  130
<212>  PRT
<213>  Artificial Sequence

<220>
<223>  Re30B06

<400>  101

Gln Val Gln Leu Val Glu Ser Gly Gly Gly Leu Val Gln Pro Gly Gly

1               5                   10                  15

Ser Leu Lys Leu Ser Cys Glu His Ser Gly Phe Thr Leu Asp Tyr Tyr
            20                  25                  30

Thr Ile Gly Trp Phe Arg Gln Ala Pro Gly Lys Glu Arg Glu Gly Val
        35                  40                  45

Gly Cys Val Gly Ser Ser Asp Gly Thr Thr Tyr Tyr Ser Asp Ser Val
    50                  55                  60

Lys Gly Arg Phe Ser Ile Ser Arg Asp Asp Ala Lys Asn Thr Val Tyr
65                  70                  75                  80

Leu Gln Met Asn Asn Leu Lys Pro Glu Asp Thr Ala Val Tyr Tyr Cys
                85                  90                  95

Ala Gly Gly Pro Gln Thr Tyr Tyr Gly Gly Arg Tyr Tyr Thr Thr Cys
            100                 105                 110

His Glu Asp Ala Met Tyr His Trp Gly Lys Gly Thr Leu Val Thr Val
        115                 120                 125

Ser Ser
    130

<210>  102
<211>  9
<212>  PRT
<213>  Artificial Sequence

<220>
<223>  Re30B06 CDR1

<400>  102

Phe Thr Leu Asp Tyr Tyr Thr Ile Gly
1               5

<210>  103
<211>  15
<212>  PRT
<213>  Artificial Sequence

<220>
<223>  Re30B06 CDR2

<400>  103

Gly Cys Val Gly Ser Ser Asp Gly Thr Thr Tyr Tyr Ser Asp Ser
1               5                   10                  15

<210>  104
<211>  22
<212>  PRT
<213>  Artificial Sequence

<220>
<223>  Re30B06 CDR3

<400>  104

Gly Gly Pro Gln Thr Tyr Tyr Gly Gly Arg Tyr Tyr Thr Thr Cys His
1               5                   10                  15

Glu Asp Ala Met Tyr His
            20

<210>  105
<211>  128
<212>  PRT
<213>  Artificial Sequence

<220>
<223>  Re32E06

<400>  105

Gln Val Gln Leu Val Glu Ser Gly Gly Gly Leu Val Gln Pro Gly Gly
1               5                   10                  15

Ser Leu Arg Leu Ser Cys Glu Thr Ser Gly Ile Thr Leu Asp Tyr Tyr
            20                  25                  30

Ala Ile Gly Trp Phe Leu Gln Ala Pro Gly Lys Glu Arg Glu Gly Val
        35                  40                  45

Ala Cys Met Arg Asn Trp Asp Gly Ser Thr Leu Tyr Ala Pro Ser Val
    50                  55                  60

Lys Gly Arg Phe Thr Ile Ser Arg Asp Asn Asp Lys Lys Ala Val Tyr
65                  70                  75                  80

Leu Glu Met Asn Ser Leu Lys Ser Glu Asp Thr Gly Val Tyr Tyr Cys
                85                  90                  95

Ala Ala Gly Pro Leu Pro Pro Gly His Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210>  106
<211>  9
<212>  PRT

<213>  Artificial Sequence

<220>
<223>  Re32E06 CDR1

<400>  106

Ile Thr Leu Asp Tyr Tyr Ala Ile Gly
1               5


<210>  107
<211>  15
<212>  PRT
<213>  Artificial Sequence

<220>
<223>  Re32E06 CDR2

<400>  107

Ala Cys Met Arg Asn Trp Asp Gly Ser Thr Leu Tyr Ala Pro Ser
1               5                   10                  15


<210>  108
<211>  20
<212>  PRT
<213>  Artificial Sequence

<220>
<223>  Re32E06 CDR3

<400>  108

Ala Gly Pro Leu Pro Pro Gly His Ser Cys Arg Ile Pro Thr Pro Leu
1               5                   10                  15


Gly Tyr Asp Asp
            20


<210>  109
<211>  128
<212>  PRT
<213>  Artificial Sequence

<220>
<223>  Ma6F06

<400>  109

Gln Val Gln Leu Val Glu Ser Gly Gly Gly Leu Val Gln Pro Gly Gly
1               5                   10                  15


Ser Leu Arg Leu Ser Cys Ala Thr Ser Gly Ile Thr Leu Asp Tyr Tyr
            20                  25                  30


Ala Ile Gly Trp Phe Leu Gln Val Pro Gly Lys Glu Arg Glu Gly Val
        35                  40                  45

Ala Cys Met Arg Asn Trp Asp Gly Ser Thr Leu Tyr Ala Pro Ser Val
    50                  55                  60

Lys Gly Arg Phe Thr Ile Ser Arg Asp Ala Asp Lys Glu Val Ala Tyr
65                  70                  75                  80

Leu Glu Met Asn Ser Leu Lys Ser Glu Asp Thr Gly Val Tyr Tyr Cys
                85                  90                  95

Ala Ala Gly Pro Leu Pro Pro Gly His Ser Cys Arg Ile Pro Thr Pro
            100                 105                 110

Leu Gly Tyr Asp Asp Trp Gly Gln Gly Thr Gln Val Thr Val Ser Ser
        115                 120                 125

<210>  110
<211>  9
<212>  PRT
<213>  Artificial Sequence

<220>
<223>  Ma6F06 CDR1

<400>  110

Ile Thr Leu Asp Tyr Tyr Ala Ile Gly
1               5

<210>  111
<211>  15
<212>  PRT
<213>  Artificial Sequence

<220>
<223>  Ma6F06 CDR2

<400>  111

Ala Cys Met Arg Asn Trp Asp Gly Ser Thr Leu Tyr Ala Pro Ser
1               5                   10                  15

<210>  112
<211>  20
<212>  PRT
<213>  Artificial Sequence

<220>
<223>  Ma6F06 CDR3

<400>  112

Ala Gly Pro Leu Pro Pro Gly His Ser Cys Arg Ile Pro Thr Pro Leu
1               5                   10                  15

Gly Tyr Asp Asp
            20

<210>  113
<211>  231
<212>  PRT
<213>  Artificial Sequence

<220>
<223>  wt_RBD-AviHis

<400>  113

Glu Thr Asn Leu Cys Pro Phe Gly Glu Val Phe Asn Ala Thr Arg Phe
1               5                   10                  15

Ala Ser Val Tyr Ala Trp Asn Arg Lys Arg Ile Ser Asn Cys Val Ala
            20                  25                  30

Asp Tyr Ser Val Leu Tyr Asn Ser Ala Ser Phe Ser Thr Phe Lys Cys
        35                  40                  45

Tyr Gly Val Ser Pro Thr Lys Leu Asn Asp Leu Cys Phe Thr Asn Val
    50                  55                  60

Tyr Ala Asp Ser Phe Val Ile Arg Gly Asp Glu Val Arg Gln Ile Ala
65                  70                  75                  80

Pro Gly Gln Thr Gly Lys Ile Ala Asp Tyr Asn Tyr Lys Leu Pro Asp
                85                  90                  95

Asp Phe Thr Gly Cys Val Ile Ala Trp Asn Ser Asn Asn Leu Asp Ser
            100                 105                 110

Lys Val Gly Gly Asn Tyr Asn Tyr Leu Tyr Arg Leu Phe Arg Lys Ser
        115                 120                 125

Asn Leu Lys Pro Phe Glu Arg Asp Ile Ser Thr Glu Ile Tyr Gln Ala
    130                 135                 140

Gly Ser Thr Pro Cys Asn Gly Val Glu Gly Phe Asn Cys Tyr Phe Pro
145                 150                 155                 160

Leu Gln Ser Tyr Gly Phe Gln Pro Thr Asn Gly Val Gly Tyr Gln Pro
                165                 170                 175

Tyr Arg Val Val Val Leu Ser Phe Glu Leu Leu His Ala Pro Ala Thr
            180                 185                 190

Val Cys Gly Pro Lys Thr Gly Thr Gly Gly Ser Gly Gly Ser Gly Leu
        195                 200                 205

Asn Asp Ile Phe Glu Ala Gln Lys Ile Glu Trp His Glu Gly Arg Thr
    210                 215                 220

Lys His His His His His His
225                 230

<210>  114
<211>  231
<212>  PRT
<213>  Artificial Sequence

<220>
<223>  TetraMutRBD-AviHis

<400>  114

Glu Thr Asn Leu Cys Pro Phe Gly Glu Val Phe Asn Ala Thr Arg Phe
1               5                   10                  15

Ala Ser Val Tyr Ala Trp Asn Arg Lys Arg Ile Ser Asn Cys Val Ala
            20                  25                  30

Asp Tyr Ser Val Leu Tyr Asn Ser Ala Ser Phe Ser Thr Phe Lys Cys
        35                  40                  45

Tyr Gly Val Ser Pro Thr Lys Leu Asn Asp Leu Cys Phe Thr Asn Val
    50                  55                  60

Tyr Ala Asp Ser Phe Val Ile Arg Gly Asp Glu Val Arg Gln Ile Ala
65                  70                  75                  80

Pro Gly Gln Thr Gly Thr Ile Ala Asp Tyr Asn Tyr Lys Leu Pro Asp
                85                  90                  95

Asp Phe Thr Gly Cys Val Ile Ala Trp Asn Ser Asn Asn Leu Asp Ser
            100                 105                 110

Lys Val Gly Gly Asn Tyr Asn Tyr Arg Tyr Arg Leu Phe Arg Lys Ser
        115                 120                 125

Asn Leu Lys Pro Phe Glu Arg Asp Ile Ser Thr Glu Ile Tyr Gln Ala
    130                 135                 140

Gly Ser Thr Pro Cys Asn Gly Val Lys Gly Phe Asn Cys Tyr Phe Pro
145                 150                 155                 160

Leu Gln Ser Tyr Gly Phe Gln Pro Thr Tyr Gly Val Gly Tyr Gln Pro

165                 170                 175

Tyr Arg Val Val Val Leu Ser Phe Glu Leu Leu His Ala Pro Ala Thr
            180                 185                 190

Val Cys Gly Pro Lys Thr Gly Thr Gly Gly Ser Gly Gly Ser Gly Leu
        195                 200                 205

Asn Asp Ile Phe Glu Ala Gln Lys Ile Glu Trp His Glu Gly Arg Thr
    210                 215                 220

Lys His His His His His His
225                 230

<210>  115
<211>  127
<212>  PRT
<213>  Artificial Sequence

<220>
<223>  Re9F06

<400>  115

Gly Ser Gln Val Gln Leu Val Glu Ser Gly Gly Gly Leu Val Gln Ala
1               5                   10                  15

Gly Gly Ser Leu Arg Leu Ser Cys Ala Ala Ser Gly Arg Thr Phe Ser
            20                  25                  30

Asn Asp Ala Leu Gly Trp Phe Arg Gln Ala Pro Arg Lys Glu Arg Glu
        35                  40                  45

Phe Val Ala Ala Ile Asn Trp Asn Ser Gly Thr Tyr Tyr Ala Asp Ser
    50                  55                  60

Val Lys Gly Arg Phe Thr Ile Ser Arg Asp Asn Ala Lys Asn Thr Val
65                  70                  75                  80

Tyr Leu Gln Met Asn Ser Leu Lys Pro Glu Asp Thr Ala Val Tyr Ser
                85                  90                  95

Cys Ala Ala Ala Ser Asp Tyr Gly Leu Pro Arg Glu Asp Phe Leu Tyr
            100                 105                 110

Asp Tyr Trp Gly Gln Gly Thr Gln Val Thr Val Ser Ser Thr Ser
        115                 120                 125

<210>  116
<211>  268

<212>  PRT
<213>  Artificial Sequence

<220>
<223>  Re9F06-Re5D06R15_Tandem

<400>  116

Glu Gly Ser Gln Val Gln Leu Val Glu Ser Gly Gly Gly Leu Val Gln
1               5                   10                  15

Ala Gly Gly Ser Leu Arg Leu Ser Cys Ala Ala Ser Gly Arg Thr Phe
            20                  25                  30

Ser Asn Asp Ala Leu Gly Trp Phe Arg Gln Ala Pro Arg Lys Glu Arg
        35                  40                  45

Glu Phe Val Ala Ala Ile Asn Trp Asn Ser Gly Thr Tyr Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Ser Cys Ala Ala Ala Ser Asp Tyr Gly Leu Pro Arg Glu Asp Phe Leu
            100                 105                 110

Tyr Asp Tyr Trp Gly Gln Gly Thr Gln Val Thr Val Ser Glu Thr Glu
        115                 120                 125

Gly Glu Gly Glu Gly Gly Gly Glu Gly Gly Glu Gly Ser Gln Val Gln
    130                 135                 140

Leu Val Glu Ser Gly Gly Gly Leu Val Glu Pro Gly Gly Ser Leu Arg
145                 150                 155                 160

Leu Ser Cys Ala Ala Ser Gly Ile Thr Leu Asp Tyr Tyr Ala Met Gly
                165                 170                 175

Trp Phe Arg Glu Ala Pro Gly Lys Glu Arg Glu Gly Val Ala Arg Ile
            180                 185                 190

Arg Asn Ser Asp Gly Ser Thr Asn Tyr Ala Asp Ser Val Lys Gly Arg
        195                 200                 205

Phe Thr Met Ser Arg Asp Asn Ala Lys Asp Thr Tyr Tyr Leu Gln Met
    210                 215                 220

Asn Ser Leu Glu Pro Glu Asp Thr Ala Asp Tyr Tyr Cys Ala Tyr Gly
225                 230                 235                 240

Pro Leu Thr Lys Tyr Gly Ser Ser Trp Tyr Trp Pro Tyr Glu Tyr Asp
                245                 250                 255

Tyr Trp Gly Glu Gly Thr Glu Val Thr Val Ser Ser
            260                 265

<210>  117
<211>  268
<212>  PRT
<213>  Artificial Sequence

<220>
<223>  Re9F06-Re5D06R28_Tandem

<400>  117

Glu Gly Ser Gln Val Gln Leu Val Glu Ser Gly Gly Gly Leu Val Gln
1               5                   10                  15

Ala Gly Gly Ser Leu Arg Leu Ser Cys Ala Ala Ser Gly Arg Thr Phe
            20                  25                  30

Ser Asn Asp Ala Leu Gly Trp Phe Arg Gln Ala Pro Arg Lys Glu Arg
        35                  40                  45

Glu Phe Val Ala Ala Ile Asn Trp Asn Ser Gly Thr Tyr Tyr Ala Asp
    50                  55                  60

Ser Val Lys Gly Arg Phe Thr Ile Ser Arg Asp Asn Ala Lys Asn Thr
65                  70                  75                  80

Val Tyr Leu Gln Met Asn Ser Leu Lys Pro Glu Asp Thr Ala Val Tyr
                85                  90                  95

Ser Cys Ala Ala Ala Ser Asp Tyr Gly Leu Pro Arg Glu Asp Phe Leu
            100                 105                 110

Tyr Asp Tyr Trp Gly Gln Gly Thr Gln Val Thr Val Ser Glu Thr Glu
        115                 120                 125

Gly Glu Gly Glu Gly Gly Gly Glu Gly Gly Glu Gly Ser Gln Val Gln
    130                 135                 140

Leu Val Glu Ser Gly Gly Gly Leu Val Glu Pro Gly Gly Ser Leu Arg
145                 150                 155                 160

Leu Ser Cys Ala Ile Ser Gly Ser Thr Leu Asp Tyr Tyr Ala Met Gly
                165                 170                 175

Trp Phe Arg Glu Ala Pro Gly Lys Glu Arg Glu Gly Val Ala Arg Trp
            180                 185                 190

Arg Asn Asn Asp Gly Ser Thr Asn Tyr Ala Asp Ser Val Lys Gly Arg
        195                 200                 205

Phe Thr Met Ser Arg Asp Asn Ala Lys Asp Thr Tyr Tyr Leu Gln Met
    210                 215                 220

Asn Ser Leu Glu Pro Glu Asp Thr Ala Asp Tyr Tyr Cys Ala Tyr Gly
225                 230                 235                 240

Pro Leu Thr Lys Tyr Gly Ser Ser Trp His Trp Pro Tyr Glu Tyr Asp
                245                 250                 255

Tyr Trp Gly Glu Gly Thr Glu Val Thr Val Ser Ser
            260                 265

## Description

### Field of the invention

The present invention pertains in the fields of antibody technology, protein engineering, medicine, pharmacology, infection biology, virology, and medical diagnostics. More specifically, the present disclosure provides VHH antibodies that prevent cell entry of and infection by SARS-CoV-2 and that have been selected for potent cross-reaction and cross-neutralization between the original Wuhan strain and the Alpha, Beta, Gamma, Delta, and Mu variants of concern.

### Background of the invention

The COVID-19 pandemics has plunged the world into a nearly unprecedented medical and economic crisis - mainly because only insufficient means for treatment are available. COVID-19 is caused by SARS-CoV-2 - a recently evolved virus belonging to the *Coronaviridae.* The virus infects the upper airways initially but can also cause pneumonia and more systemic infections with damages to lungs, kidneys and/or the circulatory system. Approximately 2% of the WHO-documented infections had a lethal outcome so far, whereby the probability of a severe course increases with the age of the individuals and with risk factors such as obesity, diabetes, and chronic lung or kidney diseases.

SARS-CoV-2 enters cells with the help of its homotrimeric spike protein, which docks to the ACE2 receptor of the host and subsequently mediates fusion between the viral and host cell membranes to allow entry of the viral genomic RNA into the now infected host cell. The host cell is then re-programmed to produce viral proteins, to replicate the viral RNA, and package replicated RNA into the next generation of viral particles, which are then shed in large numbers off the host cell to infect further cells.

The adaptive immune system can control viral infections by a T-cell mediated cytotoxic response that eliminates infected cells and/ or by B-cells producing antibodies that bind to the virus's surface and block infection (for a textbook view see: Murphy and Weaver, 2016). Such infection-blocking antibodies are referred to as neutralizing ones. Typically, however, only a small fraction of produced anti-viral antibodies also has neutralizing properties.

Immunoglobulin G (IgG) is not only the most abundant class of antibodies but also the most important one in terms of long-term immunity. IgGs are built from four polypeptide chains, two light and two heavy ones. They contain two antigen-binding sites formed each by one heavy and one light chain. Such a composite binding site can confer a virus-neutralizing activity. In addition, IgGs comprise an Fc fragment, formed only by the two heavy chains. The Fc fragment is a prototypic immune effector domain that can interact with cell surface receptors (called Fc receptors) as well as with components of the complement system.

In some cases, however, antibodies might even help viral entry by an effect called ADE (for antibody-dependent enhancement) that is mediated by Fc-receptor interactions (Kliks *et al*., 1989; Littaua *et al*., 1990). Likewise, the immune system might aggravate the condition of patients with COVID-19 by a systemic over-reaction that manifests itself as a cytokine storm (Chen *et al*., 2020; Mehta *et al*., 2020) or an inappropriate complement activation (Magro *et al*., 2020).

An effective immune response will either prevent a virus from establishing an infection in the first place or reduce, after an initial infection, the virus-load to non-pathogenic levels. An insufficiently controlled SARS-CoV-2 infection, however, might have a lethal outcome. In survivors, it often causes permanent damage (e.g., lung dysfunction) and impairs quality of life. An efficient anti-viral therapy would be highly desirable, ideally with orthogonal lines of treatment that target distinct steps of the viral infection cycle independently.

Remdesivir represents so far one line of treatment. This nucleoside analogue inhibits the SARS-CoV-2 RNA polymerase. As of May 2020, clinical studies gave mixed outcomes: from no decrease in mortality to a modest reduction in hospitalization time (Wang *et al*., 2020b).

Another treatment option for severely infected patients is passive immunization with polyclonal antibodies/blood plasma from individuals who have already recovered from COVD-19 (Duan *et al*., 2020). This approach is considered as a last resort and generally has several drawbacks, such as the risk of ADE in case of an insufficient neutralization, limited availability of sera from convalescent individuals, inevitable batch-to-batch variability of plasma samples, the risk of transmitting other infectious diseases, and all other potential adverse effects when large amounts of antibodies of mixed specificity are transferred from one person to another.

A more advanced approach is the isolation of monoclonal antibodies from SARS-CoV-2 infected or convalescent patients (Wang *et al*., 2020a). These can be characterized in-depth and produced reproducibly in a recombinant form. This solves some of the issues raised for the plasma-therapy mentioned above. However, monoclonal antibodies are very costly to produce, making it hard to imagine that it will be feasible to scale up production to levels needed for the treatment of large numbers of patients. In addition, immunological side effects, such as aggravating cytokine storms, inappropriate complement activation, or ADE, cannot be ruled out either, mainly due to the Fc region present in human/ humanized antibodies.

By now, several SARS-CoV-2 variant strains with mutated RBDs, i.e., escape mutants, have emerged that are causing devastating outbreaks. The emergence of such escape mutants is presumably driven by the selective pressure of the immune system, particularly in immunocompromised, persistently infected patients (Clark *et al*., 2021; Starr *et al*., 2021). Escape mutants can bypass antibody-based immunity in previously infected or vaccinated individuals. This has the potential of sustaining infection waves in populations that have acquired herd immunity against earlier strains.

Escape mutants also pose a tremendous challenge for therapeutic antibodies, which may be rendered ineffective by a given mutation. Indeed, several monoclonal anti-SARS-CoV-2 antibodies developed for treating COVID-19 are ineffective against the South African and Brazilian variants (Garcia-Beltran *et al*., 2021; Hoffmann *et al*., 2021; Li *et al*., 2021; Tada *et al*., 2021; Wibmer *et al*., 2021; Zhou *et al*., 2021).

Camelids are unusual in that they produce heavy chain-only antibodies, whose simpler antigen-binding domain can easily be cloned and recombinantly expressed in bacteria or yeast (Arbabi Ghahroudi *et al*., 1997). Such isolated antigen-binding domains are then referred to as nanobodies or VHH antibodies (variable domain of heavy homodimer antibodies). They are just ~14kDa in size - about one-tenth of a traditional IgG molecule. They have found numerous useful applications, for example as crystallization chaperones (Rasmussen *et al*., 2011; Chug *et al*., 2015), as tools in affinity chromatography, for localizing proteins in cells or as an animal-friendly substitute for secondary antibodies (Pleiner *et al*., 2015, 2018).

VHH antibodies recognizing the SARS-CoV-2 spike protein S1 domain are described in co-owned application PCT/EP2021/071309 the content of which is herein incorporated by reference.

It was an object of the present invention to provide VHH antibodies showing improved binding and neutralization characteristics with SARS-CoV-2 variant strains including the Delta variant.

### Summary of the invention

According to the present invention, SARS-CoV-2-neutralizing VHH antibodies are provided, which are suitable for the effective treatment of COVID-19 patients. Specifically, the invention provides VHH antibodies that neutralize virus already at a very low concentration. Further, the invention provides stable VHH antibodies.

In particular embodiments, the VHH antibodies are monomeric VHH antibodies.

In further embodiments, the VHH antibodies are multimeric VHH antibodies, particularly homotrimeric VHH antibodies or heterodimeric VHH antibodies.

A first aspect of the invention relates to an VHH antibody recognizing the SARS-CoV-2 spike protein S1 domain, particularly the receptor-binding domain (RBD) of the SARS-CoV-2 S1 domain.

The VHH antibody is selected from the group of VHH antibodies comprising a CDR3 sequence as disclosed herein or a related CDR3 sequence. In certain embodiments, the VHH antibody is selected from the group of VHH antibodies comprising a combination of CDR1, CDR2 and CDR3 sequences as disclosed herein or a related combination of CDR1, CDR2 and CDR3 sequences. In certain embodiments, the VHH antibody is selected from the group of antibodies comprising a VHH sequence as disclosed herein.

In certain embodiments, the SARS-CoV-2-neutralizing VHH antibody is a stable, particularly a thermostable or a hyperthermostable VHH antibody.

In certain embodiments, the VHH antibody as described above is covalently or non-covalently conjugated to a heterologous moiety, which may be selected from a labeling group, a capture group or an effector group.

In certain embodiments, the VHH antibody as described above is fused to a heterologous polypeptide moiety, which may be selected from a multimerization module, e.g., dimerization, trimerization or tetramerization module. In particular embodiments, the multimerization module is trimerization module.

In certain embodiments, the VHH antibody is conjugated to one or several polymer moieties, preferably hydrophilic polymer moieties, such as polyethylene glycol (PEG), to increase the molecular weight of the antibody conjugate and, thus, delay renal clearance. The molecular weight of a polymer moiety may vary over a broad range, for example in the range of about 5 kDa to about 80 kDa. Such coupling may be performed through e.g., amino or carboxyl groups already present in the VHHs and/or through the side chains of engineered lysine, aspartic acid, glutamic acid or cysteine residues and involve known chemistries for forming amide bonds, secondary amine bonds or thioether bonds.

In certain embodiments, the VHH antibody as described above is directed against the SARS-CoV-2 spike protein receptor-binding domain (RBD). In certain embodiments, the VHH antibody binds to the RBD of the SARS-CoV-2 Wuhan strain and to a tetramutant RBD of a SARS-CoV-2 having the immune-escape mutations K417T, L452R, E484K and N501Y.

In certain embodiments, the VHH antibody is capable of virus neutralization. In certain embodiments, the SARS-CoV-2-neutralizing VHH antibody neutralizes a SARS-CoV2 variant, e.g., a variant comprising at least one mutation in the RBD, particularly at position K417, L452, T478, E484 and/or Y501, more particularly at least one mutation from the group consisting of K417T, K417N, L452R, E484K, N501Y and T478K. In certain embodiments, the VHH antibody cross-neutralizes the SARS-CoV-2 Wuhan strain, the Alpha variant, the Beta variant, the Gamma variant, the Mu variant, and the Delta variant.

A further aspect of the present invention relates to a set of two or more different VHH antibodies as described above, wherein the different VHH antibodies may recognize different epitopes, particularly different epitopes on the RBD, and more particularly non-overlapping epitopes on the RBD.

A VHH antibody described above is suitable for use in medicine, e.g., human medicine, particularly for use in therapy, e.g., in the prevention or treatment of a disorder caused by and/or associated with an infection with SARS-CoV-2, or in diagnostics, e.g., for detecting SARS-CoV-2 virus or viral components in a patient sample, e.g., in a body fluid or tissue sample.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for recombinant production of a VHH antibody as described above, comprising cultivating a cell or an organism as described above in a suitable medium and obtaining the VHH antibody from the cell or organism or from the medium.

Still a further aspect of the invention relates to a method for the prevention or treatment of a disorder caused by and/or associated with an infection with SARS-CoV-2 including a SARS-CoV2 mutant, comprising administering an effective dose of the VHH antibody as described above or the set of at least two different VHH antibodies as described above to a subject in need thereof, particularly to a human subject infected with SARS-CoV-2 or at risk from being infected with SARS-CoV-2.

A list of VHH antibodies of the present invention and their utility for virus neutralization is shown in the following Table 1:

**Table 1**

| **VHH** | **Class** | **Epitope** | **Thermostability** | **RBD-Affinity (pM)** | | | **Lowest neutralizing concentration (pM)^{∗}** | | | |
|---|---|---|---|---|---|---|---|---|---|---|
| | | | | **WT** | **Tetra mutant** | **WT** | **Alpha** | **Beta** | **Gamma** | **Delta** |
| Re5D06R15 | Re5D06 | 1 | >95°C | ≤10 | ≥100 000 | 50 | 100 | >50 000 | | 1700 |
| Re6H06 | Re6H06 | 1 | >95°C | ≤10 | 3 000 | 170 | 1 700 | 1 700 | | 1 700 |
| Re9B09 | Re9B09 | 1 | | ≤10 | 25 000 | 500 | 1 700 | 3000 | | 3 000 |
| Re9H03 | Re9B09 | 1 | | 24 | 150 | 1000 | 1 700 | 700 | | 3 500 |
| Re9F06-Re5D06R15 | Tandem | 1+2 | >95°C | ≤10 | 150 | 50 | 50 | 170 | | 80 |
| Re9F06-Re5D06R28 | Tandem | 1+2 | >95°C | ≤10 | 150 | 50 | 90 | 500 | | 170 |
| Re30A03 | Re30A03 | 1 | 60°C | | ≤10 | | | 170 | | 500 |
| Re30B06 | Re30B06 | 1 | >95°C | | ≤10 | | 200 | 170 | 100 | 170 |
| Re30B09 | Re30H02 | 1 | >95°C | | ≤10 | | | 170 | | 500 |
| Re30D01 | Re30D01 | 2 | 62°C | | ≤10 | | | <500 | | <500 |
| Re30E02 | Re30E02 | 1 | >95°C | | ≤100 | | | 500 | | 500 |
| Re30H02 | Re30H02 | 1 | >95°C | | ≤100 | 50 | 170 | 50 | | 1000 |
| **Ma2H07** | **Re9B09** | **1** | **69°C** | **n.d.** | **50** | **170** | **350** | **270** | | **170** |
| Ma2E06 | Re9B09 | 1 | 74°C | 40 | 3 000 | 170 | 160 | 290 | | 300 |
| Ma2F10 | Re9B09 | 1 | 66°C | n.d. | 400 | 50 | 170 | 500 | | 500 |
| Re31A08 | (Re6H06) | 1 | >95°C | 60 | 450 | | 500 | | | 500 |
| Re31A12 | (Re6H06) | 1 | >95°C | 120 | 240 | | 170 | | | 500 |
| Re32A10 | Re32D03 | 1 | >95°C | 4 | 570 | | 50 | | | 500 |
| Re32B02 | Re32D03 | 1 | >95°C | ≤10 | 500 | | 50 | 170 | | 170 |
| Re32C07 | Ma4H03 | 1 | >95°C | ≤10 | 700 | | 170 | | | 50 |
| Re32C08 | Re32D03 | 1 | >95°C | ≤10 | 750 | | 50 | | | 170 |
| **Re32D03** | **Re32D03** | **1** | **>95°C** | **≤10** | **20** | **50** | **50** | **170** | **50** | **50** |
| Re32F02 | Re32D03 | 1 | >95°C | ≤10 | 540 | | 50 | 500 | | 170 |
| Re32G01 | Re32D03 | 1 | >95°C | 16 | 460 | | 50 | | | 170 |
| **Re32B11** | **Re32D03** | **1** | **>95°C** | | **≤10** | **50** | **50** | **400** | **50** | **170** |
| Re32D08 | Re32D03 | 1 | >95°C | | 80 | 70 | 50 | 170 | | 170 |
| Re32F03 | Re32D03 | 1 | >95°C | | 450 | 50 | 50 | 170 | | 170 |
| Re32G06 | Re32D03 | 1 | >95°C | | ≤10 | 50 | 50 | 500 | | 170 |
| Re32E06 | Re32D03 | 1 | >95°C | ≤10 | 450 | | 50 | 200 | 50 | 170 |
| Ma6F06 | Re32D03 | 1 | >95°C | ≤10 | 450 | | 50 | | | 170 |

***Table 1:** Anti-RBD VHHs, their affinities for the SARS-CoV-2 RBD (expressed* as *KD values) and neutralization potencies. Smaller numbers for affinities and neutralizing concentrations are better. *Geometric means from* ≥*2 experiments each.*

*Monomeric VHH antibodies Re5D06R15, Re6H06, ReB09, Re9H03 and heterodimeric VHH antibodies (tandems) Re9F06-Re5D06R15 and Re9F06-Re5D06R28 are described in* PCT/EP2021/071309*, supra. All other VHH antibodies are described for the first time in the present invention.*

The variable regions (CDRs) of each VHH antibody of Table 1 as well as assignment of SEQ ID NOs for VHHs and CDRs are listed in the following Table 2:

**Table 2**

| VHH | SE Q ID NO. | CDR1 | SE Q ID NO. | CDR2 | SE Q ID NO. | CDR3 | SE Q ID NO. |
|---|---|---|---|---|---|---|---|
| Re5D06 | 1 | ITLDYYAIG | 2 | SRIRSSDGSTNYADS | 3 | YGPLTKYGSSWYWPYEYDY | 4 |
| Re5D06R1 5 | 5 | ITLDYYAM G | 6 | ARIRNSDGSTNYADS | 7 | YGPLTKYGSSWYWPYEYDY | 8 |
| Re6H06 | 9 | VTLDYYAIG | 10 | SCTSSSDGSTYYADS | 11 | VVPQTYYGGKYYSQCTANGMDY | 12 |
| Re9B09 | 13 | FTLDYYAIG | 14 | SRISSSDGSTDYADS | 15 | TVPGTYYSGNWYYTWHPEAVDY | 16 |
| Re31A08 | 17 | ITLDYYSIG | 18 | SCTSSRDDSTDYADS | 19 | LTPATYYSGRYYFQCPPHGMDY | 20 |
| Re31A12 | 21 | VTLDYYAIG | 22 | SCTSSSDGSTNYSDS | 23 | VTPATYYGGRYYFQCPYHDMHY | 24 |
| Ma02H07 | 25 | ITLDYYAIG | 26 | SYISSSNDRTDYADS | 27 | TVTGTYYSGRWYYNWHPEAVDY | 28 |
| Ma02F10 | 29 | ITLDYYAIG | 30 | SYISGSNDRTDYADS | 31 | TVTGTYYSGRWYYNWHPEAVDY | 32 |
| Ma02E06 | 33 | ITLDYYAIG | 34 | SYISNTNDKTDYADS | 35 | TVPGTYYGGRWYYTWHPEAVRY | 36 |
| Re32D03 | 37 | ITLDYYAVG | 38 | SCMRNWDGRTVYAP S | 39 | AGPLPPGISCRIPTPLGYDD | 40 |
| Re32G06 | 41 | ITLDYYAVG | 42 | SCMRNWDGRTVYAP S | 43 | AGPLPPGTSCRIPTPLGYDD | 44 |
| Re32B11 | 45 | ITLDYYAIG | 46 | SCMRNSDGRTVYAP S | 47 | AGPLPPGISCRIPTPLGYDD | 48 |
| Re32A10 | 49 | ITLDYYAIG | 50 | SCMRSSDGSTVYAPS | 51 | AGPLPPGTSCRIPTPLGYDD | 52 |
| Re32B02 | 53 | ITLDYYAIG | 54 | SCMRSSDGSTVYAPS | 55 | AGPLPPGTSCRIPTPLGYDD | 56 |
| Re32F03 | 57 | ITLDYYAIG | 58 | ACMRSSDERSLYAPS | 59 | SGSVVPGTMCRIPTPLGYDD | 60 |
| Re32C08 | 61 | ITLDYYAIG | 62 | ACMRNWDGSTVYAP S | 63 | AGPLPPGHSCRIPTPLGYDD | 64 |
| Re32G01 | 65 | ITLDYYAIG | 66 | ACMRNWDGSTL YAP S | 67 | AGPLPPGHSCRIPTPLGYDD | 68 |
| Re32F02 | 69 | LTLDYYGIG | 70 | SCLGVPDNRTLYAPS | 71 | AGPVVPGHMCRIPTPLGYDY | 72 |
| Re32D08 | 73 | LTLDYYGIG | 74 | SCLGVPDNRTLYAPS | 75 | AGPVVPGHMCRIPTPLGYDY | 76 |
| Re30H02 | 77 | FTLDYYTIG | 78 | SCIGSSDSSTSYADA | 79 | AGPATYYGGNWHHTCHANAMHY | 80 |
| Re30B09 | 81 | FTLDYYTIG | 82 | SCIGSSDSSTSYADA | 83 | AGPATYYGGNWHHTCHANAMDY | 84 |
| Re30A12 | 85 | VTLDYYTIG | 86 | SCIGSGDGSTSYADA | 87 | VGPATYYGGNWHYTCHADAMRD | 88 |
| Re30D01 | 89 | PIFSFNAVD | 90 | AQIGHHGATNYVDS | 91 | AFVSINIPQGREEFGY | 92 |
| Re30A03 | 93 | VTLDYYGL C | 94 | ACISSVDGATVYADS | 95 | AGPMTPYGGRWYFSGLLAGNGMH Y | 96 |
| Re30E02 | 97 | LTLDYYTIG | 98 | TCIASSDGNTVYADS | 99 | GGPQTYYGGTYHSTCHENAMDY | 100 |
| Re30B06 | 101 | FTLDYYTIG | 102 | GCVGSSDGTTYYSDS | 103 | GGPQTYYGGRYYTTCHEDAMYH | 104 |
| Re32E06 | 105 | ITLDYYAIG | 106 | ACMRNWDGSTL YAP S | 107 | AGPLPPGHSCRIPTPLGYDD | 108 |
| Ma6F06 | 109 | ITLDYYAIG | 110 | ACMRNWDGSTL YAP S | 111 | AGPLPPGHSCRIPTPLGYDD | 112 |

***Table 2:** VHH and their CDR regions. Note that CDRs include here also variable residues that flank the actual CDR loops*

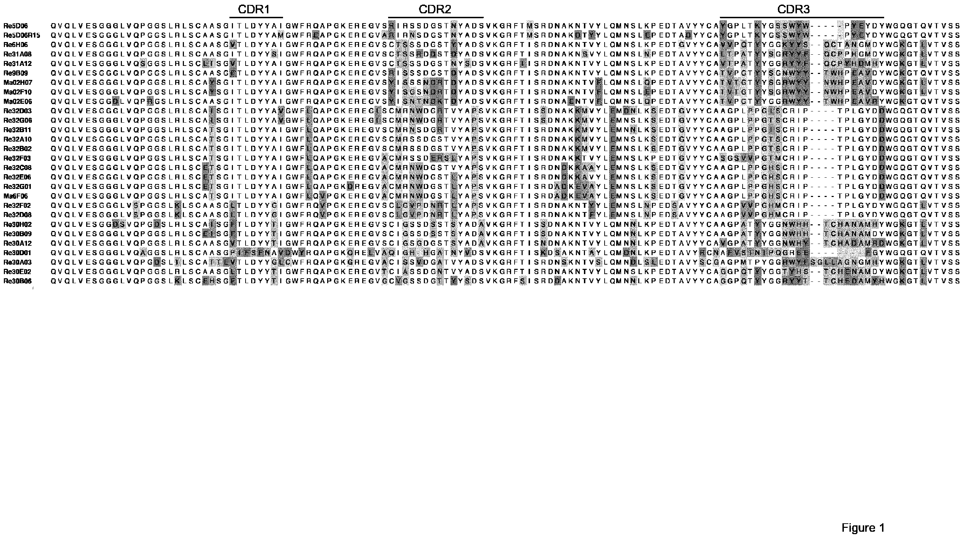

A list of complete sequences of VHH antibodies is shown at the end of the specification (see also Figure 1).

### Embodiments of the invention

In the following, specific embodiments of the invention are disclosed as follows:
1. A VHH antibody recognizing the SARS-CoV-2 spike protein S1 domain, particularly the receptor-binding domain (RBD) of the SARS-CoV-2 S1 domain comprising
   (a) a CDR3 sequence as shown in SEQ. ID NO: 20, 24, 28, 32, 36, 40, 44, 48, 52, 56, 60, 64, 68, 72, 76, 80, 84, 88, 92, 96, 100, 104,108 or 112,
   (b) a CDR3 sequence, which has an identity of at least 80%, at least 90% or at least 95% to a CDR3 sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
2. The VHH antibody according to embodiment 1 comprising
   (a) a combination of CDR1, CDR2 and CDR3 sequences as shown in SEQ. ID NO: 18-20, 22-24, 26-28, 30-32, 34-36, 38-40, 42-44, 46-48, 50-52, 54-56, 58-60, 62-64, 66-68, 70-72, 74-76, 78-80, 82-84, 86-88, 90-92, 94-96, 98-100, 102-104, 106-108, or 110-112,
   (b) a combination of CDR1, CDR2 and CDR3 sequences which has an identity of at least 80%, at least 90% or at least 95% to a combination of CDR1, CDR2 and CDR3 sequences of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
3. The VHH antibody according to embodiment 1 or 2 comprising
   (a) a VHH sequence as shown in SEQ. ID NO: 17, 21, 25, 29, 33, 37, 41, 45, 49, 53, 57, 61, 65, 69, 73, 77, 81, 85, 89, 93, 97, 101, 105, or 109,
   (b) a sequence which has an identity of at least 70%, at least 80%, at least 90%, at least 95% or at least 99% to a VHH sequence of (a), or
   (c) a VHH antibody, which competes with a VHH antibody of (a) for the binding to the SARS-CoV-2 spike protein S1 domain, particularly the receptor-binding domain (RBD) of the SARS-CoV-2 S1 domain.
4. The VHH antibody of any one of embodiments 1-3, which binds to the RBD of the SARS-CoV-2 Wuhan strain (comprised in SEQ. ID NO: 113) and to a tetramutant RBD of a SARS-CoV-2 having the mutations K417T, L452R, E484K and N501Y (comprised in SEQ. ID NO: 114) with an affinity expressed as dissociation constant KD of about 1000 pM or less, of about 500 pM or less, of about 100 pM or less or of about 50 pM or less.
5. The VHH antibody of any one of embodiments 1-4, which neutralizes SARS-CoV-2, e.g., the SARS-CoV-2 Wuhan strain, or a SARS-CoV-2 variant.
6. The VHH antibody of embodiment 5, which neutralizes a SARS-CoV2 variant comprising a spike protein RBD including at least one mutation, particularly at position K417, L452, T478, E484 and/or Y501, more particularly at least one mutation selected from the group consisting of K417T, K417N, L452R, E484K, N501Y and T478K.
7. The VHH antibody of embodiment 5 or 6, which neutralizes a variant of concern including Alpha (N501Y), Beta (N501Y, E484K, K417N), Gamma (N501Y, E484K, K417T), Delta (L452R, T478K), Epsilon (L452R), and/or Mu (E484K, N501Y).
8. The VHH antibody of any one of embodiments 5-7, which neutralizes SARS-CoV-2 or a SARS-CoV-2 variant at a concentration of about 5 nM or less, of about 1000 pM or less, of about 500 pM or less, of about 170 pM or less, or of about 100 pM or less.
9. The VHH antibody of any one of embodiments 5-8, which cross-neutralizes the SARS-CoV-2 Wuhan strain, the Alpha variant, the Beta variant, the Gamma variant, and the Delta variant, particularly at a concentration of about 5 nM or less, of about 1000 pM or less, of about 500 pM or less, of about 170 pM or less, or of about 100 pM or less.
10. The VHH antibody of any one of embodiments 1-9, which is stable, particularly thermostable or hyperthermostable.
11. The VHH antibody of embodiment 10, which has a melting temperature of at least about 65°C, of at least about 80°C, of at least 90°C or of at least about 95°C when measured under non-reducing conditions.
12. The VHH antibody of embodiment 10 or 11, which has an aggregation temperature of at least about 50°C, of at least about 60°C, of at least 70°C or of at least about 80°C when measured under non-reducing conditions.
13. The VHH antibody of any one of embodiments 1-12, which is selected from antibody Re32D03 comprising a VHH sequence as shown in SEQ. ID NO: 37 or a VHH antibody, which is a variant thereof.
14. The VHH antibody of any one of embodiments 1-12, which is selected from antibody Re32B11 comprising a VHH sequence as shown in SEQ. ID NO: 45 or a VHH antibody, which is a variant thereof.
15. The VHH antibody of any one of embodiments 1-12, which is selected from antibody Re30D01 comprising a VHH sequence as shown in SEQ. ID NO: 89 or a VHH antibody, which is a variant thereof.
16. The VHH antibody of any one of embodiments 1-12, which is selected from antibody Re32E06 comprising a VHH sequence as shown in SEQ. ID NO: 105 or a VHH antibody, which is a variant thereof.
17. The VHH antibody of any one of embodiments 1-12, which is selected from antibody Ma2H07 comprising a VHH sequence as shown in SEQ. ID NO: 25 or a VHH antibody, which is a variant thereof.
18. The VHH antibody of any one of embodiments 1-17, which is non-glycosylated.
19. The VHH antibody of any one of embodiments 1-18, which is produced in a bacterium, e.g., *E. coli.*
20. The VHH antibody of any one of embodiments 1-18, which is produced in a yeast, e.g., *Pichia pastoris.*
21. The VHH antibody of any one of embodiments 1-20, which is a monomeric VHH antibody.
22. The VHH antibody of any one of embodiments 1-20, which is a multimeric VHH antibody.
23. The VHH antibody of embodiment 22, which is a homo-multimeric, e.g., a homotrimeric VHH antibody comprising 3 subunits, wherein each subunit comprises a monomeric VHH antibody fused to a trimerization module, e.g., a collagen trimerization moiety, particularly a human collagen moiety, or a lung surfactant protein D moiety.
24. The VHH antibody of embodiment 22, which is a heterodimeric VHH antibody, particularly a covalently linked VHH heterodimer comprising a first VHH antibody and a second VHH antibody wherein the first VHH antibody and the second VHH antibody bind to different epitopes on the RBD of the SARS-CoV-2 S1 domain.
25. The VHH antibody of embodiment 24, which comprises two VHH antibodies that bind to non-overlapping RBD epitopes.
26. The VHH antibody of any one of embodiments 1-25, which is covalently or non-covalently conjugated to a heterologous moiety, e.g., a labeling group, a capture group or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.
27. The VHH antibody of any one of embodiments 1-26, which is fused to a heterologous polypeptide moiety.
28. The VHH antibody of any of embodiments 1-27, which is conjugated to one or several polymer moieties, preferably hydrophilic polymer moieties, such as polyethylene glycol (PEG).
29. A set of two or more different VHH antibodies recognizing the SARS-CoV-2 spike protein S1 domain, particularly the receptor-binding domain (RBD) of the SARS-CoV-2 S1 domain, comprising at least one VHH antibody of any of embodiments 1-28, particularly a monomeric VHH antibody of embodiment 19.
30. The set of embodiment 29, wherein the different VHH antibodies recognize different epitopes on the RBD, particularly non-overlapping epitopes on the RBD.
31. The VHH antibody of any one of embodiments 1-28 or the set of embodiment 29 or 30 for use in medicine, e.g., human medicine, particularly for use in therapy or diagnostics.
32. The VHH antibody of any one of embodiments 1-28 or the set of embodiment 29 or 30 for use in the prevention or treatment of a disorder caused by and/or associated with an infection with SARS-CoV-2 including an infection with a SARS-CoV-2 escape mutant.
33. The VHH antibody of any one of embodiments 1-28 or the set of embodiment 29 or 30 for use in the prevention or treatment of COVID-19 in a human subject.
34. The VHH antibody of any one of embodiments 1-28 or the set of embodiment 29 or 30 for use in the detection of SARS-CoV-2 virus.
35. Use of the VHH antibody of any one of embodiments 1-28 or the set of embodiment 29 or 30 for detecting SARS-CoV-2 virus or viral components in a patient sample, e.g., in a body fluid or tissue sample.
36. Use of the VHH antibody of any one of embodiments 1-28 or the set of embodiment 29 or 30 for detecting SARS-CoV-2 virus or viral components in a virus culture, e.g., in a culture of SARS-CoV-2 or variants thereof including pseudo typed variants, or in a genetically modified organism, e.g., in an organism producing viruses or viral components.
37. The use of embodiment 36 for monitoring, quantification and/or quality control during production of viruses or viral components.
37. A nucleic acid molecule encoding a VHH antibody or a subunit of a VHH antibody according to any one of embodiments 1-28, preferably in operative linkage with a heterologous expression control sequence.
38. A vector comprising a nucleic acid molecule according to embodiment 37.
39. A recombinant cell or non-human organism transformed or transfected with a nucleic acid molecule according to embodiment 37 or a vector according to embodiment 38.
40. The cell or organism of embodiment 39, which is selected from a bacterium such as E. *coli Bacillus sp.,* a unicellular eukaryotic organism, e.g., yeast such as *Pichia pastoris,* or *Leishmania,* an insect cell, a mammalian cell or a plant cell.
41. A method for recombinant production of a VHH antibody of any one of embodiments 1-28, comprising cultivating a cell or an organism of embodiment 39 or 40 in a suitable medium and obtaining the VHH antibody from the cell or organism or from the medium.
42. The method of embodiment 41, comprising cultivating a yeast such as *Pichia pastoris* and obtaining the VHH antibody from the medium.
43. A method for the prevention or treatment of a disorder caused by and/or associated with an infection with SARS-CoV-2, comprising administering an effective dose of the VHH antibody of any one of embodiments 1-28 or the set of embodiment 29 or 30 to a subject in need thereof, particularly to a human subject.

### Detailed description of the invention

The primary aim of this aspect of the invention has been to generate, select, engineer and optimize SARS-CoV-2-neutralising VHH antibodies for effective treatment of COVID-19 patients. Specifically, we aimed for VHH antibodies that are capable of virus neutralization of SARS-CoV-2 variants, particularly SARS-CoV-2 variants of concerns and of cross-neutralization of the original SARS-CoV-2 Wuhan strain and SARS-CoV-2 variants already at a very low concentration. Further, we aimed for VHH antibodies that have sufficient stability for pharmaceutical applications.

According to the present invention, VHH antibodies are provided which cross-neutralize the SARS-CoV-2 Wuhan strain and variants of concern at very low concentrations.

In previous work, e.g., as described in PCT/EP2021/071309, supra, VHH antibodies from three alpacas immunized with SARS-CoV-2 S1 and RBD were obtained including neutralizing VHH antibodies directed against two RBD epitopes, namely epitope 1 (the main epitope defined by VHH antibody Re5D06 shown SEQ ID NO: 1) and epitope 2 (the epitope defined by the VHH antibody Re9F06 shown in SEQ ID NO: 115). Epitope 1 has a very large overlap with ACE2, epitope 2 only a small overlap. Epitope 1-binders such as Re5D06 were found to be more potent in neutralization (neutralizing down to concentrations of 50 pM) than epitope 2 binders (so far not better than 1.7 nM). However, epitope 1 was found to be mutated in all variants of concern, namely in Alpha (N501Y), Beta (N501Y, E484K, K417N), Gamma (N501Y, E484K, K417T), Delta (L452R, T478K), Epsilon (L452R) and Mu (E484K, N501Y). These escape mutations impede binding and neutralization by epitope 1-binding VHH antibodies, rendering them less suitable in therapeutic applications.

As a solution to the problem, the inventors repeatedly re-immunized the same three alpacas with wildtype and mutant spike proteins, allowing further affinity maturation of antibodies and their adaptation to RBD mutations. Immune libraries were prepared, and phage display performed, cross-selecting with alternating RBD versions as a bait, namely with a "wildtype" RBD (comprised in SEQ. ID NO: 113) corresponding to the original Wuhan strain and a "tetramutant" RBD (comprised in SEQ. ID NO: 114) combining the four key immune escape mutations K417T, L452R, E484K, and N501Y) found in the Alpha, Beta, Gamma, Delta, Epsilon and Mu SARS-CoV-2 strains. The phage display also comprised a selection against non-neutralizing VHH-antibodies, using an RBD-ACE2 fusion as a competitor that blocks VHHs that do not compete with ACE2 for RBD-binding.

This combined strategy yielded new VHH antibodies (Tables 1 and 2). These include VHH variants of previously isolated classes (e.g., belonging to the Re9B09 (SEQ: ID NO: 13) class), mostly however, new VHH classes (notably the Re32D03, and the Re30H01 classes).

### VHH antibodies

The present invention relates to a VHH antibody, which is a monovalent heavy chain-only antibody comprising a CDR1 domain, a CDR2 domain and a CDR3 domain linked by framework regions including, but not being limited to, whole VHH antibodies, e.g. native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing. According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which competes with a specific VHH antibody disclosed herein for the binding to the SARS-CoV-2 spike protein S1 domain. In certain embodiments, a competing VHH antibody binds the same or an overlapping epitope on the SARS-CoV-2 spike protein S1 domain, particularly the receptor-binding domain (RBD) of the SARS-CoV-2 S1 domain as a specific VHH antibody disclosed herein. For example, the invention refers to a VHH antibody, which competes either with VHH antibodies Re32D03, Re32B11, Re32E06, or Re30D01, or with VHH antibody Ma2H07.

Competition can be measured either as an at least 90% loss of fluorescence staining of the SARS-CoV-2 spike protein by a fluorophore-labelled candidate VHH antibody in the presence of a 100-fold molar excess of an unlabelled competitor VHH antibody, e.g., selected from Re32D03, Re32B11, Re32E06, Re30D01, or Ma2H07, or conversely as an at least 90% loss of spike protein-staining by a fluorophore-labelled VHH antibody in the presence of a 100-fold molar excess of the unlabelled candidate VHH antibody as a competitor.

Preferably, competition can be measured by label-free biolayer interferometry - performed as a cross-competition or epitope binning assay using a label-free detection system, e.g., an Octet^{®} system from Sartorius, according to the manufacturer's instructions.

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In further particular embodiments, the VHH antibody is selected from antibody Re32D03 comprising a VHH sequence as shown in SEQ. ID NO: 37 or a VHH antibody, which is a variant thereof. In certain embodiments, 1, 2, 3, 4, 5, 6, 7, 8, 9 or 10 amino acids of SEQ. ID NO: 37 are replaced by another amino acid.

In further particular embodiments, the VHH antibody is selected from antibody Re32E06 comprising a VHH sequence as shown in SEQ. ID NO: 105 or a VHH antibody, which is a variant thereof. In certain embodiments, 1, 2, 3, 4, 5, 6, 7, 8, 9 or 10 amino acids of SEQ. ID NO: 105 are replaced by another amino acid.

In further particular embodiments, the VHH antibody is selected from antibody Re32B11 comprising a VHH sequence as shown in SEQ. ID NO: 45 or a VHH antibody, which is a variant thereof. In certain embodiments, 1, 2, 3, 4, 5, 6, 7, 8, 9 or 10 amino acids of SEQ. ID NO: 45 are replaced by another amino acid.

In further particular embodiments, the VHH antibody is selected from antibody Re30D01 comprising a VHH sequence as shown in SEQ. ID NO: 89 or a VHH antibody, which is a variant thereof. In certain embodiments, 1, 2, 3, 4, 5, 6, 7, 8, 9 or 10 amino acids of SEQ. ID NO: 89 are replaced by another amino acid.

In further particular embodiments, the VHH antibody is selected from antibody Ma2H07 comprising a VHH sequence as shown in SEQ. ID NO: 25 or a VHH antibody, which is a variant thereof. In certain embodiments, 1, 2, 3, 4, 5, 6, 7, 8, 9 or 10 amino acids of SEQ. ID NO: 25 are replaced by another amino acid.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an E. coli or a Bacillus sp. cell, a yeast cell, particularly a *Pichia* yeast cell, an insect cell or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

### Binding to the SARS-CoV-2 RBD

The VHH antibodies of the present invention bind to the S1 domain, particularly to the RBD of the S1 domain of SARS-CoV-2. The inventors have identified VHH antibodies which bind with high affinity to the RBD of the SARS-CoV-2 Wuhan strain and to a tetramutant RBD of a SARS-CoV-2 having the mutations K417T, L452R, E484K and N501Y as shown in Table 1, supra.

In certain embodiments, the VHH antibody of the present invention binds to the RBD of the SARS-CoV-2 Wuhan strain (comprised in SEQ. ID NO: 113) and to a tetramutant RBD of a SARS-CoV-2 having the mutations K417T, L452R, E484K and N501Y (comprised in SEQ. ID NO: 114) with an affinity expressed as dissociation constant KD of about 1000 pM or less, of about 500 pM or less, of about 100 pM or less or of about 50 pM or less. The binding affinity may be determined as described herein in detail below.

### Virus neutralization

The VHH antibodies of the present invention are capable of neutralizing SARS-CoV-2, e.g., the SARS-CoV-2 Wuhan strain, or a SARS-CoV-2 variant. The inventors have identified VHH antibodies which cross-neutralize the SARS-CoV-2 Wuhan strain, the Alpha variant, the Beta variant, the Gamma variant, and the Delta variant, as shown in Table 1, supra.

In certain embodiments, the VHH antibody of the present invention neutralizes SARS-CoV-2, e.g., the SARS-CoV-2 Wuhan strain, or a SARS-CoV-2 variant at a concentration of about 5 nM or less, of about 1000 pM or less, of about 500 pM or less of about 170 pM or less, or of about 100 pM or less. The neutralization potency may be determined as described herein in detail below.

In particular embodiments, the VHH antibodies are capable of neutralizing a SARS-CoV2 variant comprising a spike protein RBD including at least one mutation, particularly at position K417, L452, T478, E484 and/or Y501, more particularly at least one mutation selected from the group consisting of K417T, K417N, L452R, E484K, N501Y and T478K. In even more particular embodiments, the VHH antibodies are capable of neutralizing a SARS-CoV-2 variant of concern including Alpha (N501Y), Beta (N501Y, E484K, K417N), Gamma (N501Y, E484K, K417T), Delta (L452R, T478K), Epsilon (L452R) and/or Mu (E484K, N501Y).

In further particular embodiments, the VHH antibodies are capable of cross-neutralizing the SARS-CoV-2 Wuhan strain, and at least one of the Alpha variant, the Beta variant, the Gamma variant, and the Delta variant. In further particular embodiments, the VHH antibodies are capable of cross-neutralizing the SARS-CoV-2 Wuhan strain, the Alpha variant, the Beta variant, the Gamma variant, and the Delta variant. In even more particular embodiments, the VHH antibodies are capable of cross-neutralizing the SARS-CoV-2 Wuhan strain, the Alpha variant, the Beta variant, the Gamma variant, and the Delta variant at a concentration of about 5 nM or less, of about 1000 pM or less, of about 170 pM or less, or of about 100 pM or less. The neutralization potency may be determined as described herein in detail below.

In particular embodiments, the VHH antibodies neutralize the Delta variant of SARS-CoV-2 a concentration of about 5 nM or less, of about 1000 pM or less, of about 170 pM or less, or of about 100 pM or less, or of about 50 pM or less.

### Stability

For the intended therapeutic application, the anti-SARS-CoV-2 VHH antibodies should not only be highly potent in virus neutralization, but also, they should be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry. The present inventors have identified several thermostable or hyperthermostable VHH antibodies as shown in Table 1, supra.

In a particular embodiment, the invention relates to a VHH antibody, which is stable, particularly thermostable or hyperthermostable. Preferably, the VHH antibody has a melting point (melting temperature) of at least about 65°C, of at least about 80°C, of at least 90°C or of at least about 95°C, and/or an aggregation temperature of at least about 50°C, of at least about 60°C, of at least 70°C or of at least about 80°C when measured under non-reducing conditions. Melting and aggregation temperatures are determined as described herein.

The hyperthermostable VHH antibodies includes some extremely strong RBD-binders and very potent neutralizers, such as Re32D03 and Re32B11 (neutralization of the Delta variant at 50 or 170 pM).

### Sets of VHH antibodies

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1. In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

### Heterodimeric VHH antibodies

In certain embodiments, the VHH antibody is a heterodimeric VHH antibody, particularly a covalently linked VHH heterodimer comprising a first VHH antibody and a second VHH antibody wherein the first VHH antibody and the second VHH antibody bind to different epitopes on the RBD of the SARS-CoV-2 S1 domain.

A heterodimeric VHH antibody comprises two different VHH antibodies optionally connected by linker. In particular embodiments, at least one of the VHH antibodies is a VHH antibody of the present invention as described herein. A VHH antibody of a heterodimeric VHH antibody is characterized by (i) a CDR3 sequence, (ii) a combination of a CDR1 sequence, a CDR2 sequence, and a CDR3 sequence, (iii) by a complete VHH sequence, or (iv) by competition with a specific reference antibody.

In certain embodiments, the first and the second VHH antibody do not compete with each other as described above. The first and the second VHH antibody may be directly fused to each other or fused through a linker sequence. In particular embodiments, the first and the second VHH antibody are genetically fused by a peptidic linker sequence.

In certain embodiments, the linker is a peptidic linker, particularly having a length of 5 to 50 amino acid residues, particularly of 10 to 40 amino acid residues and more particularly of 12 to 30 amino acid residues, particularly as described in PCT/EP2021/071309 supra.

### Homotrimeric VHH antibodies

In certain embodiments, the VHH antibody is a homotrimeric antibody.

Homotrimerization of a VHH antibody requires an appropriate polypeptide fusion partner as trimerization module. According to the present invention, a fusion partner is preferred, comprising at least one of the following features:

Preferred trimerization modules include domains derived from collagen, the most abundant class of proteins of the human body, including the trimerization (NC1) domains of collagen XV (PDB 3N3F, 54 residues) (Wirz *et al*., 2011), collagen XVIII (PDB 3HSH; 54 residues) (Boudko *et al*., 2009) and collagen X (PDB 1GR3; 160 amino acids) (Bogin *et al*., 2002). Another very appropriate homotrimeric fusion partner is the lung surfactant protein D (LSFPD) that recognizes sugars on viral and bacterial pathogens (see e.g., PDB 1B08, 3IKN, 3IKQ, 3IKR). As the SARS-CoV-2 spike protein is heavily glycosylated, a VHH-LSPD fusion could confer a bimodal inhibition of spike function.

According to the present invention, it is preferred that the VHH antibody is linked to the trimerization domain via a spacer having a length of at least about 5 amino acids, particularly about 5 amino acids to about 100 amino acids, and more particularly of about 10 to about 50 amino acids, particularly as described in PCT/EP2021/071309 supra.

It should be note that different arrangements for the subunits of homotrimeric VHH antibodies are possible. As just described, the trimerization module might be located N-terminally, followed by the spacer and the VHH antibody. Alternatively, the VHH antibody might be located N-terminally, followed by spacer and trimerization module. The best arrangement will depend on the geometry of the target to be neutralized.

In particular embodiments, the present invention relates to a neutralizing homotrimeric VHH antibody, e.g., a VHH antibody which has a lowest SARS-CoV-2 neutralizing concentration of about 100 pM or less, of about 25 pM or less, of about 17 pM or less, of about 10 pM or less, of about 5 pM or less or of about 1.7 pM or less.

### Production of VHH antibodies

VHH antibodies including monomeric, heterodimeric and homomultimeric, particularly homotrimeric VHH antibodies may be produced as described in PCT/EP2021/071309 supra.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The nucleic acid molecule may encode a monomeric VHH antibody or a subunit of a homomultimeric VHH antibody, particularly of a homotrimeric VHH antibody.

Any therapeutic application of neutralizing anti-SARS-CoV-2 VHH antibodies requires their production at a large scale. In this aspect, production in bacteria or yeast, e.g., *Pichia pastoris, Saccharomyces cerevisiae,* or *Hansenula polymorpha,* may be preferred. Production in yeast, e.g., *Pichia pastoris, Saccharomyces cerevisiae,* or *Hansenula polymorpha,* is particularly preferred because it ensures a quantitative formation of disulfide bonds, allows for excellent yields (in the range of grams per liter culture), while the secretion into the medium allows the subsequent purification to begin with only few contaminating proteins.

In a particular embodiment, the VHH antibody is recombinantly produced in a bacterium, e.g., *E. coli* or *Bacillus.* For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In a further particular embodiment, the VHH antibody is recombinantly produced in a eukaryotic host cell or host organism, preferably in yeast, e.g., in *Pichia pastoris, Saccharomyces cerevisiae,* or *Hansenula polymorpha.* For example, expression in a eukaryotic host cell or host organism, e.g., yeast may involve cytoplasmic and/or periplasmic expression and purification of the VHH antibody from the host cell, or preferably secretion from the host cell and purification of the VHH antibody from the culture medium. In certain embodiments, the nucleic acid sequence encoding the VHH antibody is fused to at least one sequence directing the expression into the culture medium.

The VHH antibody encoding nucleic acid molecule may comprise a sequence encoding a monomeric VHH antibody, a sequence encoding a heterodimeric VHH antibody or a sequence encoding a subunit of a multimeric VHH antibody, particularly of a trimeric VHH antibody. The sequence encoding a heterodimeric VHH antibody may comprise two sequences each encoding a monomeric VHH antibody arranged in tandem fused to each other optionally through a linker. The subunit sequence may comprise (from N-terminus to C-terminus) the VHH antibody sequence, optionally a spacer, and a multimerization domain, particularly a trimerization domain, e.g., the Collagen XVIII NC1 domain. Alternatively, the subunit sequence may comprise (from N-terminus to C-terminus) a multimerization domain, particularly a trimerization domain, optionally a spacer and the VHH antibody sequence.

### Therapeutic applications

Still a further aspect of the present invention is the use of an active agent as described above selected from a monomeric VHH antibody, a heterodimeric VHH antibody, a set of at least 2 different VHH antibodies, or a multimeric, particularly a trimeric VHH antibody in medicine, particularly for therapeutic and/or in vitro or vivo diagnostic use. In certain embodiments, the monomeric VHH antibody, the heterodimeric VHH antibody, the set of VHH antibodies or the multimeric, particularly trimeric VHH antibody is used in human medicine.

In particular embodiments, a therapeutic VHH antibody is selected from (i) a VHH antibody comprising the CDR3 sequence of the hyperthermostable VHH antibody Re32D03 or a related sequence, (ii) a VHH antibody comprising the CDR1, CDR2 and CDR3 sequences of the hyperthermostable VHH antibody Re32D03 or related sequences, (iii) a VHH antibody comprising the VHH sequence of the hyperthermostable VHH antibody Re32D03 or a related sequence, or (iv) a VHH antibody competing with VHH antibody Re32D03. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the hyperthermostable VHH antibody Re32E06 or a related sequence, (ii) a VHH antibody comprising the CDR1, CDR2 and CDR3 sequences of the hyperthermostable VHH antibody Re32E06 or related sequences, (iii) a VHH antibody comprising the VHH sequence of the hyperthermostable VHH antibody Re32E06 or a related sequence, or (iv) a VHH antibody competing with VHH antibody Re32E06. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the VHH antibody Re30D01 or a related sequence, (ii) a VHH antibody comprising the CDR1, CDR2 and CDR3 sequences of the VHH antibody Re30D01 or related sequences, (iii) a VHH antibody comprising the VHH sequence of the VHH antibody ReRe30D01 or a related sequence, or (iv) a VHH antibody competing with VHH antibody Re30D01. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the hyperthermostable VHH antibody Re32B11 or a related sequence, (ii) a VHH antibody comprising the CDR1, CDR2 and CDR3 sequences of the hyperthermostable VHH antibody Re32B11 or related sequences, (iii) a VHH antibody comprising the VHH sequence of the hyperthermostable VHH antibody Re32B11 or a related sequence, or (iv) a VHH antibody competing with VHH antibody Re32B11. In certain embodiments, 1, 2, 3, 4, 5, 6, 7, 8, 9 or 10 amino acids of the VHH sequence of the reference antibody are replaced by another amino acid.

In particular embodiments, a therapeutic VHH antibody is selected from (i) a VHH antibody comprising the CDR3 sequence of the VHH antibody Ma2H07 or a related sequence, (ii) a VHH antibody comprising the CDR1, CDR2 and CDR3 sequences of the thermostable VHH antibody Ma2H07 or related sequences, (iii) a VHH antibody comprising the VHH sequence of the thermostable VHH antibody Ma2H07 or a related sequence, or (iv) a VHH antibody competing with VHH antibody Ma2H07. In certain embodiments, 1, 2, 3, 4, 5, 6, 7, 8, 9 or 10 amino acids of the VHH sequence of the reference antibody are replaced by another amino acid.

Therapeutic applications of an active agent as described above, e.g., a monomeric VHH antibody, a heterodimeric VHH antibody, a set of at least 2 different VHH antibodies, or a multimeric, particularly trimeric VHH antibody include methods for preventing or treating a disorder caused by and/or associated with a Coronavirus infection, particularly a disorder caused by and/or associated with a SARS-CoV-2 infection and more particularly of preventing or treating COVID-19. The therapeutic applications also include methods for preventing or treating a disorder caused by and/or associated with an infection by a SARS-CoV-2 variant including the Alpha variant, the Beta variant, the Gamma variant, the Delta variant and the Epsilon variant as well as variants comprising at least one of the amino acid substitutions in the RBD domain in any one of the above variants.

In therapeutic applications, the active agent is administered in an effective amount to a subject in need thereof, particularly to a human subject. The dose will depend on the specific type of agent, e.g. monovalent VHH antibody or multimeric VHH antibody, and the type of disease.

In the prevention or treatment of COVID-19, a daily dose of a monomeric VHH antibody for a human subject is any suitable dose based on the severity of the disease, the specific type of agent, and the mode of administration, e.g., locally, or systemically, particularly from about 5 µg to about 5000 mg per day.

In the prevention or treatment of COVID-19, a daily dose of a heterodimeric VHH antibody for a human subject is any suitable dose based on the severity of the disease, the specific type of agent, and the mode of administration, e.g., locally, or systemically, particularly from about 5 µg to about 5000 mg per day.

In the prevention or the treatment of COVID-19, a daily dose of a trimeric VHH antibody for a human subject is any suitable dose based on the severity of the disease, the specific type of agent, and the mode of administration, e.g., locally or systemically, particularly from about 1 µg to about 2500 mg per day.

In the prevention or treatment of COVID-19, a unit dose of a monomeric VHH antibody for a human subject is any suitable dose based on the severity of the disease, the specific type of agent, and the mode of administration, e.g., locally, or systemically, particularly from about 5 µg to about 5000 mg per unit.

In the prevention or treatment of COVID-19, a unit dose of a heterodimeric VHH antibody for a human subject is any suitable dose based on the severity of the disease, the specific type of agent, and the mode of administration, e.g., locally, or systemically, particularly from about 5 µg to about 5000 mg per unit.

In the prevention or the treatment of COVID-19, a unit dose of a trimeric VHH antibody for a human subject is any suitable dose based on the severity of the disease, the specific type of agent, and the mode of administration, e.g., locally, or systemically, particularly from about 1 µg to about 2500 mg per unit.

Typically, the VHH antibody is administered as a pharmaceutical composition comprising the active agent and a pharmaceutically acceptable carrier or excipient.

Examples of suitable carriers and excipients for formulating antibodies or antibody fragments are well known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g., by subcutaneous, intramuscular or intravenous injection or by infusion. In further embodiments, the pharmaceutical composition may be administered locally, e.g., orally, nasally or intrapulmonary, for example by inhalation as an aerosol.

In certain embodiments, the pharmaceutical composition is administered in an early infection stage, e.g., in an infection stage where the upper airways such as the oral cavity, the nasal cavity, the paranasal sinus, the pharynx and/or the throat are infected, but the lower airways such as the bronchi and/or the lung are not infected. In further embodiments, the pharmaceutical composition is administered in a late infection stage wherein the lower airways such as the bronchi and/or the lung are infected. Further, the pharmaceutical composition may be administered in an infection stage where the subject suffers from a respiratory dysfunction and optionally is ventilated. In still further embodiments, the pharmaceutical composition may be administered prophylactically to a subject being at risk of a Coronavirus infection, e.g., a subject, which has previously, for example, within 1, 2, 3, 4, or 5 days or even longer, been in close contact with an infected subject.

The VHH antibody may be administered alone or together with a further active agent, which may be selected from anti-viral agents such as remdesivir, methotrexate, Molnupiravir, or Paxlovid (Wang *et al*., 2020b; Malone and Campbell, 2021; Stegmann *et al*., 2021).

A further aspect that needs to be considered is the plasma half-life of a VHH antibody. Proteins smaller than 40 kDa are subject to considerable renal clearance, and an isolated VHH antibody (~15 kDa) shows, therefore, a plasma half-life of only 4 hours (Hoefman *et al*., 2015). Thus, the administration might comprise repeated daily injections or continuous perfusion during anti-viral therapy that might last for days to weeks. One solution to this problem is to increase the size of the protein. Fusion to spacer and trimerization module increases the size to ≥70 kDa, and such fusion should then have a far longer plasma half-life that is no longer limited by renal clearance. One can, however, also imagine alternative application routes. In fact, inhalation of an anti-SARS-CoV-2 VHH antibody formulation would bring the therapeutic agent directly to the site of the highest and most problematic virus load, namely the airway epithelia.

The here outlined antiviral clinical strategy will become more robust if not just one but an entire set of several VHH antibodies is deployed. First, one should expect synergistic effects for neutralizing antibodies that bind to non-overlapping sites. Second, patients might develop anti-idiotypic antibodies against a given therapeutic VHH antibody and render it ineffective in treatment. A switch to another VHH would solve the problem. Third, and perhaps most importantly there is the clear risk that SARS-CoV2 evolves further, that escape mutations in its RBD might destroy the epitope of a given VHH antibody and render such VHH inefficient in therapy. This risk can be minimized by utilizing a panel of neutralizing antibodies that recognize non-identical and possibly even non-overlapping epitopes, or by at least switching to another VHH that still neutralizes a given escape mutant.

### Diagnostic applications

Diagnostic applications include in vitro methods wherein a VHH antibody is used for detecting Coronavirus spike protein in a sample, e.g., in a body fluid such as saliva, blood, serum or plasma, stool samples or in a tissue or biopsy sample. Diagnostic applications further include in vivo methods wherein a VHH antibody is used for detecting Coronavirus spike protein in a subject, particularly in a human patient. For diagnostic applications the VHH antibody carry a label for direct detection or used in combination with secondary detection reagents, e.g., antibodies, including conventional antibodies or VHH antibodies, for indirect detection according to established techniques in the art.

Further, the present invention is explained in more detail by the following Figures and Examples.

### Figure legends

### Figure 1: Sequence alignment and highlighting of variable regions

An alignment of VHH sequences from Table 2 is shown. Residues that deviate from the consensus are shown in colour. Re5D06, Re5D06R25, Re6H06 and Re9B09 have been reported before (cf. PCT/EP2021/071309) and are shown as references. Sequences are sorted according to classes. The three variable CDR regions are indicated. These comprise predicted CDR loop regions plus flanking variable residues.

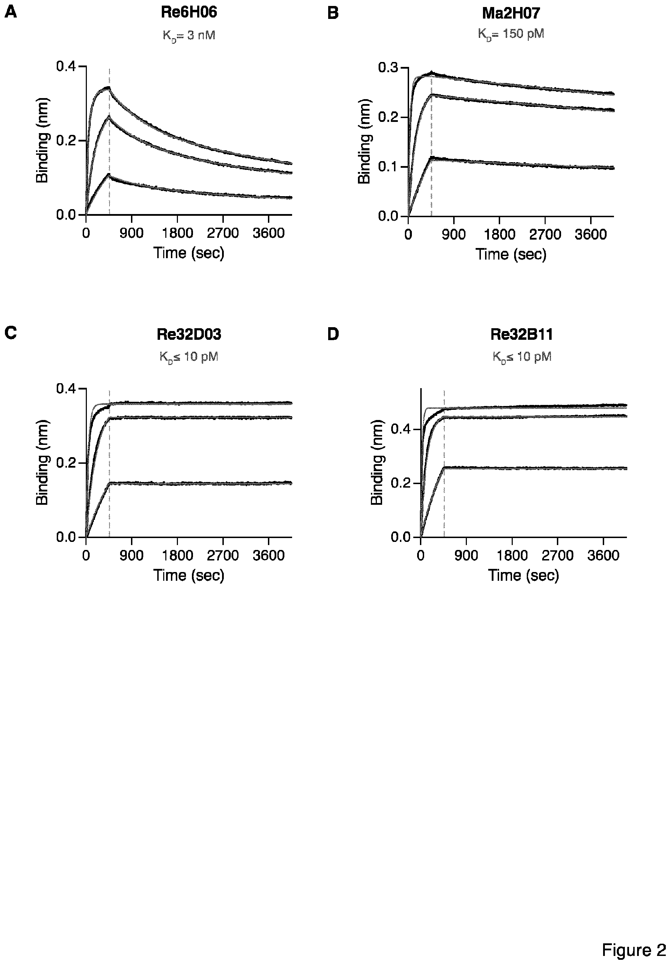

### Figure 2: Affinity measurement of VHH antibodies against a tetramutant RBD

Bio-layer interferometry (BLI) experiments were performed for the indicated VHH antibodies with a biotinylated tetramutant (K417T, L452R, E484K and N501Y) RBD. Curves (black) were fitted with a mass transport model. Fitted curves and calculated dissociation constants KDs are shown in red. red). Re6H06 (a previously isolated mutation-tolerant anti-RBD VHH) bound with a KD of 3 nM. Ma2H07 showed with a K_{D} of 150 pM a far better affinity. Re32D03 and Re32B11 had a low picomolar affinity with a non-detectable dissociation rate.

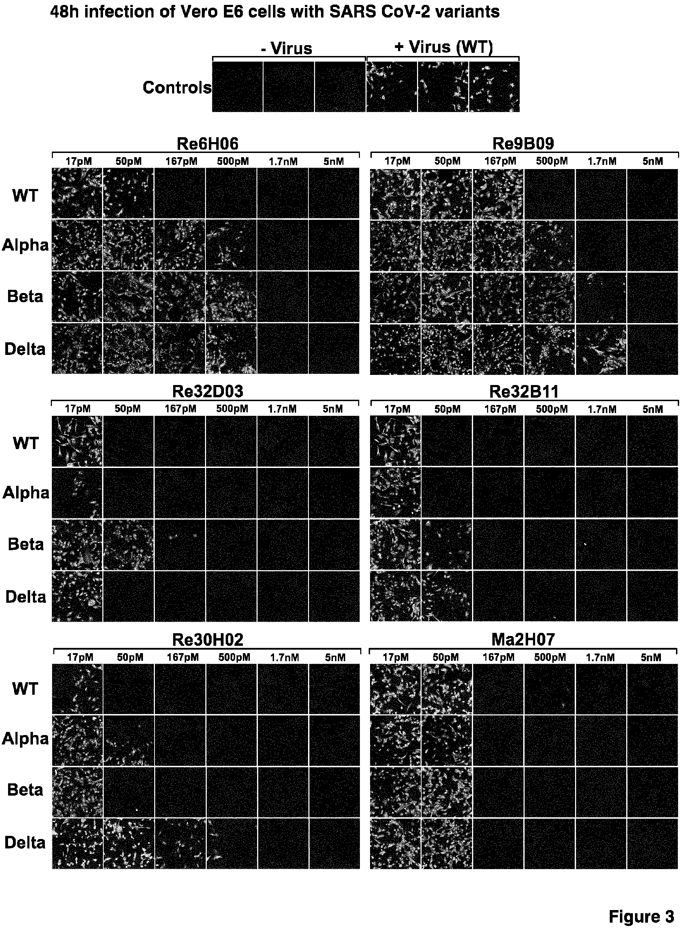

### Figure 3: Neutralization potency of VHH antibodies against SARS-CoV-2 wildtype and variants

Vero E6 cells were grown in 96 well plates and infected with the indicated SARS-CoV-2 variants. Cells were fixed 48 h later, permeabilized and stained with DAPI (to locate cell nuclei) and a cocktail of fluorescently labelled VHH antibodies that recognize three non-overlapping epitopes on the SARS-CoV-2 Spike protein. Analysis was by automated spinning disc fluorescence microscopy. Panels depict maximum intensity projections. Infected cells show bright intracellular signals in the green (anti-RBD) and red (anti-S1ΔRBD) channels. Where indicated, virus was pre-incubated with indicated concentrations of VHH antibodies.

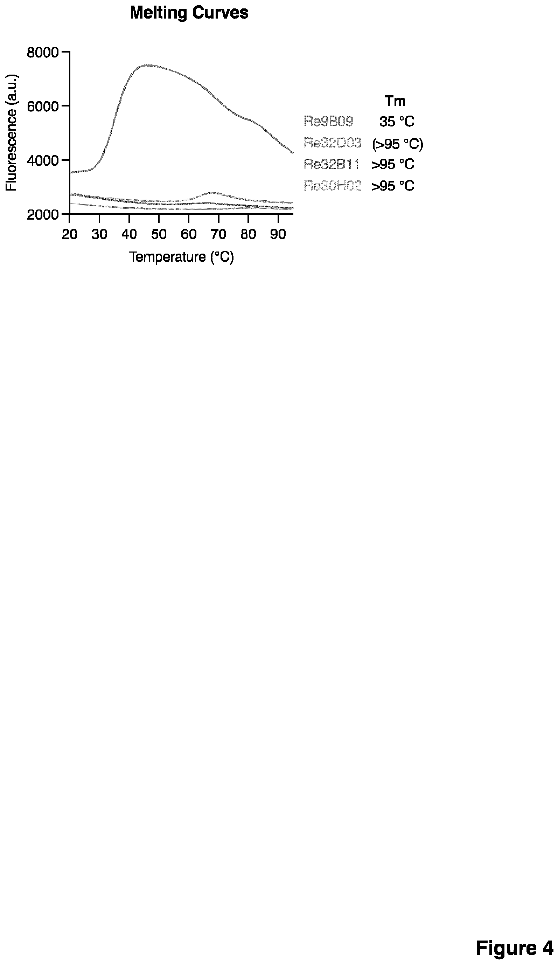

### Figure 4: Thermostability of VHH antibodies

The indicated VHH antibodies were subjected to Differential scanning fluorimetry (DSF), which exploits that thermal unfolding exposes aromatic/hydrophobic residues, which then bind and enhance fluorescence of the added SYPRO orange dye. Two replicates of each sample were placed onto a Thermal Cycler. The samples were incubated for 5 min at 25 °C and then the temperature was increased in 1 °C increments of 45 seconds to 95 °C. Fluorescence was measured at the end of each step.

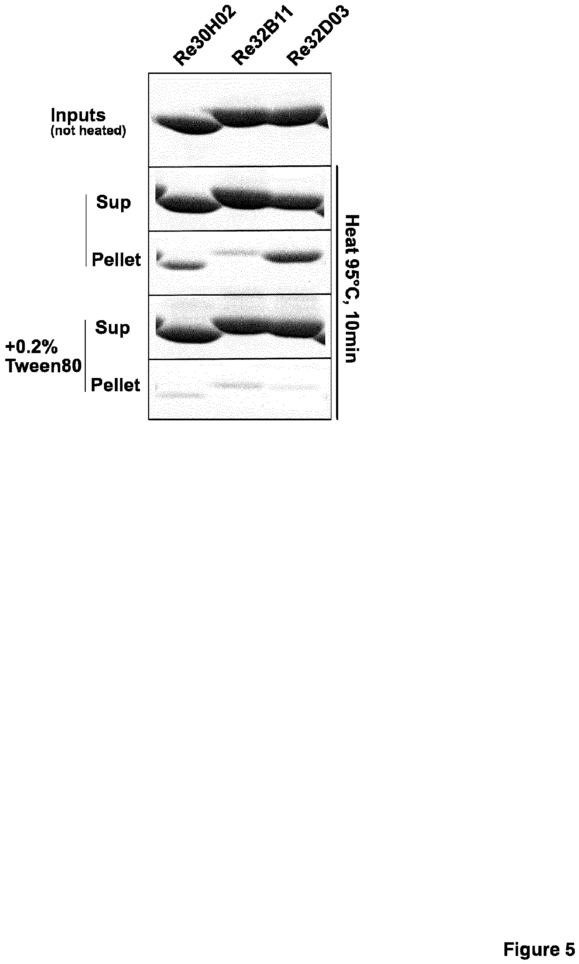

### Figure 5: Resistance to heat-induced aggregation.

The indicated VHH antibodies were diluted to a concentration of 1 mg/ml. One set of samples was supplemented with Tween 80. Then, samples were heated for 10 minutes to 95°C and centrifuged. Soluble (Sup) and precipitated fractions (Pellet) were analyzed by SDS-PAGE followed by Coomassie-staining.

### VHH antibody sequences (shown in Table 2)

>Re5D06 (SEQ ID NO. 1) 
QVQLVESGGGLVQPGGSLRLSCAASGITLDYYAIGWFRQAPGKEREGVSRIRSSD
GSTNYADSVKGRFTMSRDNAKNTVYLQMNSLKPEDTAVYYCAYGPLTKYGSSWY
WPYEYDYWGQGTQVTVSS
>Re5D06R15 (SEQ ID NO. 5) 
QVQLVESGGGLVQPGGSLRLSCAASGITLDYYAMGWFREAPGKEREGVARIRNSD
GSTNYADSVKGRFTMSRDNAKDTYYLQMNSLEPEDTADYYCAYGPLTKYGSSWY
WPYEYDYWGQGTQVTVSS
>Re6H06 (SEQ ID NO. 9) 
QVQLVESGGGLVQPGGSLRLSCAASGVTLDYYAIGWFRQAPGKEREGVSCTSSS
DGSTYYADSVKGRFTISRDNAKNTVYLQMNSLKPEDTGVYYCAVVPQTYYGGKYY
SQCTANGMDYWGKGTLVTVSS
>Re9B09 (SEQ ID NO. 13) 
QVQLVESGGGLVQPGGSLRLSCAASGFTLDYYAIGWFRQAPGKEREGVSRISSSD
GSTDYADSVKGRFTISRDNAKNTVYLQMNSLKPEDTAVYYCATVPGTYYSGNWYY
TWHPEAVDYWGKGTQVTVSS
>Re31A08 (SEQ ID NO. 17) 
QVQLVESGGGLVQPGGSLRLSCAASGITLDYYSIGWFRQAPGKEREGVSCTSSRD
DSTDYADSVKGRFTISRDNAKNSVYLQMNSLNPEDTAVYYCALTPATYYSGRYYFQ
CPPHGMDYWGKGTLVTVSS
>Re31A12 (SEQ ID NO. 21) 
QVQLVESGGGLVQSGGSLRLSCLTSGVTLDYYAIGWFRQAPGKEREGVSCTSSSD
GSTNYSDSVKGRFIISRDNAKNTVYLQMNSLKPEDTAVYYCAVTPATYYGGRYYFQ
CPYHDMHYWGKGTLVTVSS
>Ma02H07 (SEQ ID NO. 25) 
QVQLVESGGGLVQPGGSLRLSCAYSGITLDYYAIGWFRQAPGKEREGVSYISSSN
DRTDYADSVKGRFTISRDNAKNTVFLQMNSLQPEDTAVYYCATVTGTYYSGRWYY
NWHPEAVDYWGKGTLVTVSS
>Ma02F10 (SEQ ID NO. 29) 
QVQLVESGGGLVQPGGSLRLSCAYSGITLDYYAIGWFRQAPGKEREGVSYISGSN
DRTDYADSVKGRFTISRDNAKNTVFLQMNSLEPEDTAVYYCATVTGTYYSGRWYY
NWHPEAVDYWGKGTLVTVSS
>Ma02E06 (SEQ ID NO. 33) 
QVQLVESGGDLVQPRGSLRLSCAASGITLDYYAIGWFRQAPGKEREGVSYISNTND
KTDYADSVKGRFTISRDNAENTVFLQMNSLQPEDTAVYYCATVPGTYYGGRWYYT
WHPEAVRYWGKGTQVTVSS
>Re32D03 (SEQ ID NO. 37) 
QVQLVESGGGLVQPGGSLRLSCAISGITLDYYAVGWFLQAPGKEREGISCMRNWD
GRTVYAPSVKGRFTISSDNAKKMVYLEMDNLKSEDTGVYYCAAGPLPPGISCRIPT
PLGYDDWGQGTQVTVSS
>Re32G06 (SEQ ID NO. 41) 
QVQLVESGGGLVQPGGSLRLSCAISGITLDYYAVGWFLQAPGKEREGISCMRNWD
GRTVYAPSVKGRFTISSDNAKKMVYLEMNSLKSEDTGVYYCAAGPLPPGTSCRIPT
PLGYDDWGQGTQVTVSS
>Re32B11 (SEQ ID NO. 45) 
QVQLVESGGGLVQPGGSLRLSCATSGITLDYYAIGWFLQAPGKEREGVSCMRNSD
GRTVYAPSVKGRFTISRDNAKKMVYLEMNNLKSEDTGVYYCAAGPLPPGISCRIPT
PLGYDDWGQGTQVTVSS
>Re32A10 (SEQ ID NO. 49) 
QVQLVESGGGLVQPGGSLRLSCATSGITLDYYAIGWFLQAPGKEREGVSCMRSSD
GSTVYAPSVKGRFTISRDNAKKMVYLEMNSLKSEDTGVYYCAAGPLPPGTSCRIPT
PLGYDDWGQGTQVTVSS
>Re32B02 (SEQ ID NO. 53) 
QVQLVESGGGLVQPGGSLRLSCATSGITLDYYAIGWFLQAPGKEREGVSCMRSSD
GSTVYAPSVKGRFTISRDNAKKMVYLEMNSLKSEDTGVYYCAAGPLPPGTSCRIPT
PLGYDDWGQGTQVTVSS
>Re32F03 (SEQ ID NO. 57) 
QVQLVESGGGLVQPGGSLRLSCATSGITLDYYAIGWFLQAPGKEREGVACMRSSD
ERSLYAPSVKGRFTISRDNAKKTVYLEMNSLKPEDTGVYYCASGSVVPGTMCRIPT
PLGYDDWGQGTQVTVSS
>Re32C08 (SEQ ID NO. 61) 
QVQLVESGGGLVQPGGSLRLSCETSGITLDYYAIGWFLQAPGKEREGVACMRNW
DGSTVYAPSVKGRFTISRDNDKKAAYLEMNSLKSEDTGVYYCAAGPLPPGHSCRIP
TPLGYDDWGQGTQVTVSS
>Re32G01 (SEQ ID NO. 65) 
QVQLVESGGGLVQPGGSLRLSCETSGITLDYYAIGWFLQAPGKDREGVACMRNW
DGSTLYAPSVKGRFTISRDADKEVAYLEMNSLKSEDTGVYYCAAGPLPPGHSCRIP
TPLGYDDWGQGTQVTVSS
>Re32F02 (SEQ ID NO. 69) 
QVQLVESGGGLVSPGGSLKLSCAASGLTLDYYGIGWFRQVPGKEREGVSCLGVPD
NRTLYAPSVKGRFTISRDNAKNTYYLEMNSLNPEDSAVYYCAAGPVVPGHMCRIPT
PLGYDYWGQGTLVTVSS
>Re32D08 (SEQ ID NO. 73) 
QVQLVESGGGLVSPGGSLKLSCAASGLTLDYYGIGWFRQVPGKEREGVSCLGVPD
NRTLYAPSVKGRFTISRDNAKNTFYLEMNSLNPEDSAVYYCAAGPVVPGHMCRIPT
PLGYDYWGQGTQVTVSS
>Re30H02 (SEQ ID NO. 77) 
QVQLVESGGDSVQPGDSLRLSCAISGFTLDYYTIGWFRQAPGKEREGVSCIGSSD
SSTSYADAVKGRFTISSDNAKNTVYLQMNNLKPEDTAVYYCAAGPATYYGGNWHH
TCHANAMHYWGKGTLVTVSS
>Re30B09 (SEQ ID NO. 81) 
QVQLVESGGGLVQPGGSLRLSCEISGFTLDYYTIGWFRQAPGKEREGVSCIGSSD
SSTSYADAVKGRFTISSDNAKNTVYLQMNNLKPEDTAVYYCAAGPATYYGGNWHH
TCHANAMDYWGKGTLVTVSS
>Re30A12 (SEQ ID NO. 85) 
QVQLVESGGGLVQPGGSLRLSCAASGVTLDYYTIGWFRQAPGKEREGVSCIGSGD
GSTSYADAVKGRFTISNDNAKNTVYLQMNNLKPEDTAVYYCAVGPATYYGGNWHY
TCHADAMRDWGKGTLVTVSS
>Re30D01 (SEQ ID NO. 89) 
QVQLVESGGGLVQAGGSLRLSCAASGPIFSFNAVDWYRQAPGKQRELVAQIGHH
GATNYVDSVKGRFTISKDSAKNTAYLQMDNLKPEDTAVYRCNAFVSINIPQGREEF
GYWGQGTQVTVSS
>Re30A03 (SEQ ID NO. 93) 
QVQLVESGGGLVQPGDSLTLSCATTEVTLDYYGLCWFRQAPGKGREGVACISSVD
GATVYADSVKGRFTISRDNSKNTVSLQMNDLSLEDTAVYSCGAGPMTPYGGRWYF
SGLLAGNGMHYWGKGTLVTVSS
>Re30E02 (SEQ ID NO. 97) 
QVQLVESGGGLVQPGGSLRLSCAASGLTLDYYTIGWFRQAPGKEREGVTCIASSD
GNTVYADSVKGRFTISRDNAKNTVYLQMNSLKPEDTAVYYCAGGPQTYYGGTYHS
TCHENAMDYWGKGTLVTVSS
>Re30B06 (SEQ ID NO. 101) 
QVQLVESGGGLVQPGGSLKLSCEHSGFTLDYYTIGWFRQAPGKEREGVGCVGSS
DGTTYYSDSVKGRFSISRDDAKNTVYLQMNNLKPEDTAVYYCAGGPQTYYGGRYY
TTCHEDAMYHWGKGTLVTVSS
>Re32E06 SEQ ID NO. 105) 
QVQLVESGGGLVQPGGSLRLSCETSGITLDYYAIGWFLQAPGKEREGVACMRNW
DGSTLYAPSVKGRFTISRDNDKKAVYLEMNSLKSEDTGVYYCAAGPLPPGHSCRIP
TPLGYDDWGQGTQVTVSS
>Ma6F06 (SEQ ID NO. 109)

QVQLVESGGGLVQPGGSLRLSCATSGITLDYYAIGWFLQVPGKEREGVACMRNW
DGSTLYAPSVKGRFTISRDADKEVAYLEMNSLKSEDTGVYYCAAGPLPPGHSCRIP
TPLGYDDWGQGTQVTVSS

### RBD Sequences

>wt_RBD-AviHis (SEQ ID NO. 113) 
ETNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNLDS
KVGGNYNYLYRLFRKSNLKPFERDISTEIYQAGSTPCNGVEGFNCYFPLQSYGFQP
TNGVGYQPYRVVVLSFELLHAPATVCGPKTGTGGSGGSGLNDIFEAQKIEWHEGR
TKHHHHHH
>TetraMutRBD-AviHis (SEQ ID NO. 114)

ETNLCPFGEVFNATRFASVYAWNRKRISNCVADYSVLYNSASFSTFKCYGVSPTKL
NDLCFTNVYADSFVIRGDEVRQIAPGQTGTIADYNYKLPDDFTGCVIAWNSNNLDS
KVGGNYNYRYRLFRKSNLKPFERDISTEIYQAGSTPCNGVKGFNCYFPLQSYGFQP
TYGVGYQPYRVVVLSFELLHAPATVCGPKTGTGGSGGSGLNDIFEAQKIEWHEGR
TKHHHHHH

### Additional VHH sequences

>Re9F06 (SEQ. ID NO: 115) 
GSQVQLVESGGGLVQAGGSLRLSCAASGRTFSNDALGWFRQAPRKEREFVAAIN
WNSGTYYADSVKGRFTISRDNAKNTVYLQMNSLKPEDTAVYSCAAASDYGLPRED
FLYDYWGQGTQVTVSSTS
>Re9F06-Re5D06R15 Tandem (SEQ ID NO. 116) 
EGSQVQLVESGGGLVQAGGSLRLSCAASGRTFSNDALGWFRQAPRKEREFVAAI
NWNSGTYYADSVKGRFTISRDNAKNTVYLQMNSLKPEDTAVYSCAAASDYGLPRE
DFLYDYWGQGTQVTVSETEGEGEGGGEGGEGSQVQLVESGGGLVEPGGSLRLS
CAASGITLDYYAMGWFREAPGKEREGVARIRNSDGSTNYADSVKGRFTMSRDNAK
DTYYLQMNSLEPEDTADYYCAYGPLTKYGSSWYWPYEYDYWGEGTEVTVSS
> Re9F06-Re5D06R28 Tandem (SEQ ID NO. 117)

EGSQVQLVESGGGLVQAGGSLRLSCAASGRTFSNDALGWFRQAPRKEREFVAAI
NWNSGTYYADSVKGRFTISRDNAKNTVYLQMNSLKPEDTAVYSCAAASDYGLPRE
DFLYDYWGQGTQVTVSETEGEGEGGGEGGEGSQVQLVESGGGLVEPGGSLRLS
CAISGSTLDYYAMGWFREAPGKEREGVARWRNNDGSTNYADSVKGRFTMSRDN
AKDTYYLQMNSLEPEDTADYYCAYGPLTKYGSSWHWPYEYDYWGEGTEVTVSS

### Examples

### Example 1 - Periplasmic expression and purification of VHH antibody monomers

Monomeric VHH antibodies were expressed with an N-terminal DsbA signal sequence and a C-terminal His12 tag from a Kan-ColE1 plasmid harboring a T5/lac promoter in *E. coli* NEBExpress (New England Biolabs). A 125-ml pre-culture in Terrific Broth (TB) containing 50 µg/ml Kanamycin was grown overnight at 28 °C to early stationary phase. The culture was then diluted with fresh medium (500 ml, pre-warmed to 37 °C). After 30 minutes of growth at 37 °C, protein expression was induced with 0.1 mM IPTG and growth was continued for 2 hours at 37 °C, whereby the culture reached a final OD₆₀₀ of ~8. Bacteria were harvested by centrifugation and lysed by osmotic shock lysis: cell pellets were resuspended in 14ml 130 mM Tris/HCI pH 8.0, 10 mM EDTA, and sucrose was immediately added to 20% (w/v). After gentle mixing at 23 °C for 30 min, four volumes of ice-cold water were added and mixing was continued at 4 °C for 30 min. 20 mM Tris/HCI pH 7.5, 50 mM NaCl and 20 mM imidazole were added to the cell suspension. Periplasmic extract was then recovered as the supernatant of two consecutive centrifugation steps at 4 °C: a low-speed spin at 4000 xg (20 min, F13 rotor, Thermo Fisher Scientific) and a high-speed spin at 38000 rpm (~1 hour, T647.5 rotor). The VHH antibody was purified at 4 °C via Ni²⁺ EDTA-amide chelate affinity chromatography (1 ml matrix). Beads were washed with ten column volumes of 50 mM Tris/HCI pH 7.5, 300 mM NaCl, 20 mM imidazole, 0.2% (w/v) Triton X-100 and ten column volumes of buffer lacking detergent. After elution with 50 mM Tris/HCI pH 7.5, 300 mM NaCl, 500 mM imidazole, the buffer was exchanged to 50 mM Tris/HCI pH 7.5, 150 mM NaCl, 250 mM sucrose via a PD 10 desalting column (GE Healthcare). Aliquots were frozen in liquid nitrogen and stored at -80 °C. This expression/ purification strategy was used for all VHH antibodies containing four cysteines and thus two disulfide bonds.

### Example 2 - Cytoplasmic expression and purification of VHH monomers

VHH antibodies Re9B09, Ma2H07, Ma2F02 und Ma2E06 comprising just two cysteines and thus a single disulfide bond were produced as His14-ScSUMO fusions by cytoplasmic expression in E. *coli* NEBExpress Shuffle, which allows forming disulfide bonds in the bacterial cytoplasm. In brief, 200ml pre-cultures were grown overnight at 37°C in TB+50 µg/ml kanamycin in 5-liter flasks to early stationary phase. They were then diluted with 700ml fresh medium, shifted to 21°C and induced for 5 hours with 0.08 mM IPTG. 5 mM EDTA was added, bacteria were pelleted, resuspended in 50 mM Tris/HCI pH 7.5, 20 mM imidazole/HCI pH 7.5, 300 mM NaCl, frozen in liquid nitrogen and lysed by thawing plus sonication. Insoluble material was removed by ultracentrifugation at 38000 rpm (~1 hour, T647.5 rotor).

The supernatant was applied to a 2 ml Ni²⁺ EDTA-amide chelate column. The matrix was sequentially washed in resuspension buffer, resuspension buffer + 0.2% TritonX100, resuspension buffer + 700 mM NaCl, low salt buffer (resuspension buffer minus NaCl), and protease buffer (resuspension buffer with an imidazole concentration lowered to 10mM and supplemented with 250 mM sucrose). The VHH antibody was finally eluted by cleaving the His14-SUMO-tag using 100 nM S. *cerevisiae* Ulp1 for 2 hours at room temperature. The eluted VHH antibodies were frozen in liquid nitrogen and stored an -80°C until further use.

### Example 3 - Binding of mutation-tolerant monomeric VHHs and heterodimeric VHHs to the RBD of the SARS-CoV-2 Delta variant

Bio-layer interferometry (BLI) experiments were performed using High Precision Streptavidin biosensors and an Octet RED96e instrument (ForteBio/Sartorius) at 25°C with Phosphate-Buffered Saline (PBS) pH 7.4, 0.002% (w/v) Tween 20 and 0.1% (w/v) bovine serum albumin (BSA as assay buffer. For this, RBDs had been expressed with a C-terminal Avi-tag in mammalian cells and biotinylated enzymatically with BirA.

Biotinylated RBDs, namely wildtype RBD and a tetramutant (K417T, L452R, E484K and N501Y) RBD were immobilized on biosensors until a wavelength shift/binding signal of 1 nm was reached. The biosensors were dipped into wells containing 100, 20 or 4 nM VHH for 450 seconds association, and then incubated for one hour with assay buffer for dissociation. Data were reference-subtracted, and curves (black) were fitted with a mass transport model (Octet Data Analysis HT 12.0 software).

The results are shown in Table 1 and Figure 2.

Re6H06 (a previously isolated mutation-tolerant anti-RBD VHH) bound with a KD of 3 nM to the tetramutant RBD. Ma2H07 showed with a K_{D} of 150 pM a far better affinity. Re32D03 and Re32B11 had a low picomolar affinity with a non-detectable dissociation rate.

### Example 4 - Virus infection and neutralization assays.

Virus stocks were prepared as supernatants from Vero E6 cells infected with SARS-CoV-2 (Wuhan strain (WT with D), Alpha variant, Beta variant, Gamma variant, or Delta variant).

The virus stocks contained between 10¹¹ and10¹² copies of the virus genome per ml, as determined by reverse transcription and quantitative PCR (qRT-PCR) according to a standard protocol (Corman *et al*., 2020). Approximately 10⁷ virus copies were diluted in 100 µl of cell culture media, DMEM/ 2% FBS, in the presence or absence of varying concentrations of the VHH antibodies under study. After 60 min incubation at 37 °C, this was added to a monolayer of Vero E6 cells, i.e., 5000 cells in a well of a standard 96 well cell culture plate. Note that the inoculated amount of virus was approximately 50 times higher than needed for a complete infection of a well.

Cells were fixed 48 h later with 4% paraformaldehyde, permeabilized with 0.15% Triton X100, and stained with 2 µg/ml DAPI (to locate cell nuclei) and a cocktail of fluorescently labelled VHH antibodies that recognize three non-overlapping epitopes at the SARS-CoV-2 Spike protein (5 nM of Alexa Fluor 488-labeld Re7E02, Re9C07, Re9G12, and Re9H03 and 15 nM Re8H11-Atto 565 (cf. PCT/EP2021/071309) in PBS+ 1% BSA). Imaging was by spinning disc fluorescence microscopy. Neutralization was considered as successful if no infected cells were detectable within the well.

The results are shown in Table 1 and Figure 3. The newly identified VHH antibodies Re32D03, Re32B11, Re30H02 and Ma2H07 are highly potent in preventing infection by any of the four tested SARS-CoV-2 strains. VHHs Re5D06R15, Re6H06 and Re9B09 have been described earlier (cf. PCT/EP2021/071309) and have been tested here in parallel. They required higher concentrations for neutralization, in particular for the Alpha, Beta, Gamma, and Delta variants. Table 1 lists lowest neutralization concentrations averaged from up to nine sets of experiments.

### Example 5 - Thermostability measurement of VHH antibodies

VHH antibodies were subjected to Differential scanning fluorimetry (DSF), which exploits that thermal unfolding exposes aromatic/hydrophobic residues, which then bind and enhance fluorescence of the added SYPRO orange dye. Assays were performed in a volume of 20 µl, at 1 mg/ml VHH concentration in 50 mM Tris/HCI, 150 mM NaCl (pH 8.0 at 20°C) and 1x dye (diluted from a 5000x stock; Life Technologies). Two replicates of each sample were pipetted in a Hard-Shell^{®} 96-well plate (Bio-Rad). The plate was sealed with transparent MicroSeal^{®} 'B' Seal (Bio-Rad), briefly centrifuged to remove any air bubbles, and placed onto a CFX96 Real-Time System (C1000 Thermal Cycler, BioRad). The samples were incubated for 5 min at 25 °C and then the temperature was increased in 1 °C increments of 45 seconds to 95 °C. Fluorescence was measured at the end of each step with 532 nm excitation and a 555 nm long pass filter. Melting temperatures are defined as the inflection point of the first melting peak.

The results are shown in Table 1 and Figure 4.

VHH antibody Re9B09 that was produced in reducing cytosol and hence lacks disulphide bonds melts already at 35 °C, while VHH antibodies Re30H02, Re32D03 and Re32B11 showed only a negligible melting amplitude and thus remained stable throughout 95 °C.

### Example 6 - Resistance to heat-induced aggregation

VHH antibodies were diluted to a concentration of 1 mg/ml in 50 mM Tris/HCI pH 8.0, 150 mM NaCl. One set of samples was supplemented with 0.2% Tween 80 (polysorbate 80). 40µl were heated for 10 minutes to 95°C. Samples were then centrifuged at 21 000 g for 15 minutes, and soluble in precipitated fractions were analyzed by SDS-PAGE followed by Coomassie-staining.

The results are shown in Table 1 and Figure 5.

VHH antibody Re32B11 was fully aggregation resistant. The small extent of heat-induced aggregation of Re30H02 and Re32D03 could be fully suppressed by the addition of Tween 80.

### References

Arbabi Ghahroudi M, Desmyter A, Wyns L, Hamers R, Muyldermans S (1997) Selection and identification of single domain antibody fragments from camel heavy-chain antibodies. FEBS Lett, 414: 521-526
Bogin O, Kvansakul M, Rom E, Singer J, Yayon A, Hohenester E (2002) Insight into Schmid metaphyseal chondrodysplasia from the crystal structure of the collagen X NC1 domain trimer. Structure, 10: 165-173
Boudko SP, Sasaki T, Engel J, Lerch TF, Nix J, Chapman MS, Bächinger HP (2009) Crystal structure of human collagen XVIII trimerization domain: A novel collagen trimerization Fold. J Mol Biol, 392: 787-802
Chen X, Zhao B, Qu Y, Chen Y, Xiong J, Feng Y, Men D, Huang Q, Liu Y, Yang B, Ding J, Li F (2020) Detectable serum SARS-CoV-2 viral load (RNAaemia) is closely correlated with drastically elevated interleukin 6 (IL-6) level in critically ill COVID-19 patients. Clin Infect Dis*,*
Chug H, Trakhanov S, Hülsmann BB, Pleiner T, Görlich D (2015) Crystal structure of the metazoan Nup62•Nup58•Nup54 nucleoporin complex. Science, 350: 106-110
Clark SA, Clark LE, Pan J, Coscia A, McKay LGA, Shankar S, Johnson RI, Brusic V, Choudhary MC, Regan J, Li JZ, Griffiths A, Abraham J (2021) SARS-CoV-2 evolution in an immunocompromised host reveals shared neutralization escape mechanisms. Cell, S0092-8674(21)00355
Corman VM, Landt O, Kaiser M, Molenkamp R, Meijer A, Chu DK, Bleicker T, Brünink S, Schneider J, Schmidt ML, Mulders DG, Haagmans BL, van der Veer B, van den Brink S, Wijsman L, Goderski G, Romette JL, Ellis J, Zambon M, Peiris M, Goossens H, Reusken C, Koopmans MP, Drosten C (2020) Detection of 2019 novel coronavirus (2019-nCoV) by real-time RT-PCR. Euro Surveill, 25***:***
Duan K, Liu B, Li C, Zhang H, Yu T, Qu J, Zhou M, Chen L, Meng S, Hu Y, Peng C, Yuan M, Huang J, Wang Z, Yu J, Gao X, Wang D, Yu X, Li L, Zhang J, Wu X, Li B, Xu Y, Chen W, Peng Y, Hu Y, Lin L, Liu X, Huang S, Zhou Z, Zhang L, Wang Y, Zhang Z, Deng K, Xia Z, Gong Q, Zhang W, Zheng X, Liu Y, Yang H, Zhou D, Yu D, Hou J, Shi Z, Chen S, Chen Z, Zhang X, Yang X (2020) Effectiveness of convalescent plasma therapy in severe COVID-19 patients. Proceedings of the National Academy of Sciences, 117: 9490-9496
Garcia-Beltran WF, Lam EC, St Denis K, Nitido AD, Garcia ZH, Hauser BM, Feldman J, Pavlovic MN, Gregory DJ, Poznansky MC, Sigal A, Schmidt AG, Iafrate AJ, Naranbhai V, Balazs AB (2021) Multiple SARS-CoV-2 variants escape neutralization by vaccine-induced humoral immunity. Cell, 184: 2372-2383.e9
Hoefman S, Ottevaere I, Baumeister J, Sargentini-Maier M (2015) Pre-Clinical Intravenous Serum Pharmacokinetics of Albumin Binding and Non-Half-Life Extended Nanobodies®. Antibodies, 4: 141-156
Hoffmann M, Arora P, Groß R, Seidel A, Hörnich BF, Hahn AS, Krüger N, Graichen L, Hofmann-Winkler H, Kempf A, Winkler MS, Schulz S, Jäck HM, Jahrsdörfer B, Schrezenmeier H, Müller M, Kleger A, Munch J, Pöhlmann S (2021) SARS-CoV-2 variants B.1.351 and P.1 escape from neutralizing antibodies. Cell, 184: 2384-2393.e12
Kliks SC, Nisalak A, Brandt WE, Wahl L, Burke DS (1989) Antibody-dependent enhancement of dengue virus growth in human monocytes as a risk factor for dengue hemorrhagic fever. Am J Trop Med Hyg, 40: 444-451
Li Q, Nie J, Wu J, Zhang L, Ding R, Wang H, Zhang Y, Li T, Liu S, Zhang M, Zhao C, Liu H, Nie L, Qin H, Wang M, Lu Q, Li X, Liu J, Liang H, Shi Y, Shen Y, Xie L, Zhang L, Qu X, Xu W, Huang W, Wang Y (2021) SARS-CoV-2 501Y.V2 variants lack higher infectivity but do have immune escape. Cell, 184: 2362-2371.e9
Littaua R, Kurane I, Ennis FA (1990) Human IgG Fc receptor II mediates antibody-dependent enhancement of dengue virus infection. J Immunol, 144: 3183-3186
Magro C, Mulvey JJ, Berlin D, Nuovo G, Salvatore S, Harp J, Baxter-Stoltzfus A, Laurence J (2020) Complement associated microvascular injury and thrombosis in the pathogenesis of severe COVID-19 infection: A report of five cases. Transl Res, 220: 1-13
Malone B, Campbell EA (2021) Molnupiravir: coding for catastrophe. Nat Struct Mol Biol, 28: 706-708
Mehta P, McAuley DF, Brown M, Sanchez E, Tattersall RS, Manson JJ, HLH Across Speciality Collaboration UK (2020) COVID-19: consider cytokine storm syndromes and immunosuppression. Lancet, 395: 1033-1034
Murphy K, Weaver C. (2016) Janeway's Immunobiology. Garland Science,
Pleiner T, Bates M, Görlich D (2018) A toolbox of anti-mouse and anti-rabbit IgG secondary nanobodies. J Cell Biol, 217: 1143-1154
Pleiner T, Bates M, Trakhanov S, Lee CT, Schliep JE, Chug H, Böhning M, Stark H, Urlaub H, Görlich D (2015) Nanobodies: site-specific labeling for super-resolution imaging, rapid epitope-mapping and native protein complex isolation. Elife, 4: e11349
Rasmussen SG, Choi HJ, Fung JJ, Pardon E, Casarosa P, Chae PS, Devree BT, Rosenbaum DM, Thian FS, Kobilka TS, Schnapp A, Konetzki I, Sunahara RK, Gellman SH, Pautsch A, Steyaert J, Weis WI, Kobilka BK (2011) Structure of a nanobody-stabilized active state of the β(2) adrenoceptor. Nature, 469: 175-180
Starr TN, Greaney AJ, Addetia A, Hannon WW, Choudhary MC, Dingens AS, Li JZ, Bloom JD (2021) Prospective mapping of viral mutations that escape antibodies used to treat COVID-19. Science, 371: 850-854
Stegmann KM, Dickmanns A, Gerber S, Nikolova V, Klemke L, Manzini V, Schlösser D, Bierwirth C, Freund J, Sitte M, Lugert R, Salinas G, Meister TL, Pfaender S, Görlich D, Wollnik B, Groß U, Dobbelstein M (2021) The folate antagonist methotrexate diminishes replication of the coronavirus SARS-CoV-2 and enhances the antiviral efficacy of remdesivir in cell culture models. Virus Res, 302: 198469
Tada T, Dcosta BM, Zhou H, Vaill A, Kazmierski W, Landau NR (2021) Decreased neutralization of SARS-CoV-2 global variants by therapeutic anti-spike protein monoclonal antibodies. bioRxiv, 2021.02.18.431897
Wang C, Li W, Drabek D, Okba NMA, van Haperen R, Osterhaus ADME, van Kuppeveld FJM, Haagmans BL, Grosveld F, Bosch BJ (2020a) A human monoclonal antibody blocking SARS-CoV-2 infection. Nat Commun, 11: 2251
Wang Y, Zhang D, Du G, Du R, Zhao J, Jin Y, Fu S, Gao L, Cheng Z, Lu Q, Hu Y, Luo G, Wang K, Lu Y, Li H, Wang S, Ruan S, Yang C, Mei C, Wang Y, Ding D, Wu F, Tang X, Ye X, Ye Y, Liu B, Yang J, Yin W, Wang A, Fan G, Zhou F, Liu Z, Gu X, Xu J, Shang L, Zhang Y, Cao L, Guo T, Wan Y, Qin H, Jiang Y, Jaki T, Hayden FG, Horby PW, Cao B, Wang C (2020b) Remdesivir in adults with severe COVID-19: a randomised, double-blind, placebo-controlled, multicentre trial. Lancet, 395: 1569-1578
Wibmer CK, Ayres F, Hermanus T, Madzivhandila M, Kgagudi P, Oosthuysen B, Lambson BE, De Oliveira T, Vermeulen M, Van der Berg K (2021) SARS-CoV-2 501Y. V2 escapes neutralization by South African COVID-19 donor plasma. Nature medicine, 27: 622-625
Wirz JA, Boudko SP, Lerch TF, Chapman MS, Bächinger HP (2011) Crystal structure of the human collagen XV trimerization domain: A potent trimerizing unit common to multiplexin collagens. Matrix Biology, 30: 9-15
Zhou D, Dejnirattisai W, Supasa P, Liu C, Mentzer AJ, Ginn HM, Zhao Y, Duyvesteyn HME, Tuekprakhon A, Nutalai R, Wang B, Paesen GC, Lopez-Camacho C, Slon-Campos J, Hallis B, Coombes N, Bewley K, Charlton S, Walter TS, Skelly D, Lumley SF, Dold C, Levin R, Dong T, Pollard AJ, Knight JC, Crook D, Lambe T, Clutterbuck E, Bibi S, Flaxman A, Bittaye M, Belij-Rammerstorfer S, Gilbert S, James W, Carroll MW, Klenerman P, Barnes E, Dunachie SJ, Fry EE, Mongkolsapaya J, Ren J, Stuart DI, Screaton GR (2021) Evidence of escape of SARS-CoV-2 variant B.1.351 from natural and vaccine-induced sera. Cell, 184: 2348-2361.e6

## Claims

1. A VHH antibody recognizing the SARS-CoV-2 spike protein S1 domain, particularly the receptor-binding domain (RBD) of the SARS-CoV-2 S1 domain comprising
(a) a CDR3 sequence as shown in SEQ. ID NO: 20, 24, 28, 32, 36, 40, 44, 48, 52, 56, 60, 64, 68, 72, 76, 80, 84, 88, 92, 96, 100, 104, 108, or 112,
(b) a CDR3 sequence, which has an identity of at least 80%, at least 90% or at least 95% to a CDR3 sequence of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

2. The VHH antibody according to claim 1 comprising
(a) a combination of CDR1, CDR2 and CDR3 sequences as shown in SEQ. ID NO: 18-20, 22-24, 26-28, 30-32, 34-36, 38-40, 42-44, 46-48, 50-52, 54-56, 58-60, 62-64, 66-68, 70-72, 74-76, 78-80, 82-84, 86-88, 90-92, 94-96, 98-100, 102-104, 106-108, or 110-112,
(b) a combination of CDR1, CDR2 and CDR3 sequences which has an identity of at least 80%, at least 90% or at least 95% to a combination of CDR1, CDR2 and CDR3 sequences of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

3. The VHH antibody according to claim 1 or 2 comprising
(a) a VH sequence as shown in SEQ. ID NO: 17, 21, 25, 29, 33, 37, 41, 45, 49, 53, 57, 61, 65, 69, 73, 77, 81, 85, 89, 93, 97, 101, 105, or 109,
(b) a sequence which has an identity of at least 70%, at least 80%, at least 90%, at least 95% or at least 99% to a VHH sequence of (a), or
(c) a VHH antibody, which competes with a VHH antibody of (a) for the binding to the SARS-CoV-2 spike protein S1 domain, particularly the receptor-binding domain (RBD) of the SARS-CoV-2 S1 domain.

4. The VHH antibody of any one of claims 1-3, which binds to the RBD of the SARS-CoV-2 Wuhan strain (comprised in SEQ. ID NO: 113) and to a tetramutant RBD of a SARS-CoV-2 having the mutations K417T, L452R, E484K and N501Y (comprised in SEQ. ID NO: 114) with an affinity expressed as dissociation constant KD of about 1000 pM or less, of about 500 pM or less, of about 100 pM or less or of about 50 pM or less.

5. The VHH antibody of any one of claims 1-4, which neutralizes SARS-CoV-2, e.g., the SARS-CoV-2 Wuhan strain, and/or a SARS-CoV-2 variant, particularly a variant of concern including Alpha (N501Y), Beta (N501Y, E484K, K417N), Gamma (N501Y, E484K, K417T), Delta (L452R, T478K), Epsilon (L452R) and/or Mu (E484K, N501Y) at a concentration of about 5 nM or less, of about 1000 pM or less, of about 500 pM or less, of about 170 pM or less, or of about 100 pM or less.

6. The VHH antibody of claim 5, which cross-neutralizes the SARS-CoV-2 Wuhan strain, the Alpha variant, the Beta variant, the Gamma variant, the Delta variant, and the Mu variant, particularly at a concentration of about 5 nM or less, of about 1000 pM or less, of about 500 pM or less, of about 170 pM or less, or of about 100 pM or less.

7. The VHH antibody of any one of claims 1-6, which has a melting temperature of at least about 65°C, of at least about 80°C, of at least 90°C or of at least about 95°C when measured under non-reducing conditions, and/or which has an aggregation temperature of at least about 50°C, of at least about 60°C, of at least 70°C or of at least about 80°C when measured under non-reducing conditions

8. The VHH antibody of any one of claims 1-7, which is selected from:
(i) VHH antibody Re32D03 comprising a VHH sequence as shown in SEQ. ID NO: 37 or a VHH antibody, which is a variant thereof,
(ii) VHH antibody Re32E06 comprising a VHH sequence as shown in SEQ. ID NO: 105 or a VHH antibody, which is a variant thereof,
(iii) VHH antibody Re30D01 comprising a VHH sequence as shown in SEQ. ID NO: 89 or a VHH antibody, which is a variant thereof,
(iv) VHH antibody Re32B11 comprising a VHH sequence as shown in SEQ. ID NO: 45 or a VHH antibody, which is a variant thereof, and
(v) VHH antibody Ma2H07 comprising a VHH sequence as shown in SEQ. ID NO: 25 or a VHH antibody, which is a variant thereof.

9. The VHH antibody of any one of claims 1-8, which is a monomeric VHH antibody.

10. The VHH antibody of any one of claims 1-8, which is a multimeric VHH antibody, e.g., a homotrimeric VHH antibody or a heterodimeric VHH antibody.

11. The VHH antibody of any one of claims 1-10, which is covalently or non-covalently conjugated to a heterologous moiety, e.g., a labeling group, a capture group or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.

12. A set of two or more different VHH antibodies recognizing the SARS-CoV-2 spike protein S1 domain, particularly the receptor-binding domain (RBD) of the SARS-CoV-2 S1 domain, comprising at least one VHH antibody of any of claims 1-11, particularly a monomeric VHH antibody of claim 9.

13. The VHH antibody of any one of claims 1-11 or the set of claim 12 for use in medicine, e.g., human medicine, particularly for use in therapy or diagnostics.

14. The VHH antibody of any one of claims 1-11 or the set of claim 12 for use in the prevention or treatment of a disorder caused by and/or associated with an infection with SARS-CoV-2 including an infection with a SARS-CoV-2 escape mutant, particularly for use in the prevention or treatment of COVID-19 in a human subject.

15. A method for the prevention or treatment of a disorder caused by and/or associated with an infection with SARS-CoV-2, comprising administering an effective dose of the VHH antibody of any one of claims 1-11 or the set of claim 12 to a subject in need thereof, particularly to a human subject.
